# EUROPEAN PATENT SPECIFICATION

(11) **EP 4 200 316 B1**
(45) Date of publication and mention of the grant of the patent: **29.01.2025**
(21) Application number: 21759121.3
(22) Date of filing: 20.08.2021
(51) Int. Cl.: C07K 14/005, C12N 15/86, C12N 15/10

(54) **PROCESS FOR MAKING A RECOMBINANT AAV LIBRARY**
VERFAHREN ZUR HERSTELLUNG EINER REKOMBINANTEN AAV-BIBLIOTHEK
PROCÉDÉ DE FABRICATION D'UNE BIBLIOTHÈQUE DE VAA RECOMBINANT

(30) Priority: 21.08.2020 GB 202013058; 09.07.2021 GB 202109962
(43) Date of publication of application: 28.06.2023
(73) Proprietor: Oxford Genetics Limited, Oxford OX4 4HG (GB); Oxford University Innovation Limited, Oxford, OX2 0JB (GB)
(72) Inventor: CAWOOD, Ryan, Oxford, Oxfordshire OX4 4HG (GB); SU, Weiheng, Oxford, Oxfordshire OX4 4HG (GB); PAYNE, Thomas Augustus, Oxford, Oxfordshire OX4 4HG (GB)
(74) Representative: Dehns
(86) International application number: PCT/GB2021/052160
(87) International publication number: WO 2022/038367

(56) References cited:
- WO-A1-2019/020992
- WO-A2-2004/020600
- MÜLLER O J ET AL: "Random peptide libraries displayed on adeno-associated virus to select for targeted gene therapy vectors", NATURE BIOTECHNOLOGY, GALE GROUP INC, NEW YORK, vol. 21, no. 9, 1 September 2003 (2003-09-01), pages 1040 - 1046, XP002596751, ISSN: 1087-0156, [retrieved on 20030803], DOI: 10.1038/NBT856
- WATERKAMP D A ET AL: "Isolation of targeted AAV2 vectors from novel virus display libraries", THE JOURNAL OF GENE MEDICINE, JOHN WILEY & SONS, INC, US, vol. 8, no. 11, 1 November 2006 (2006-11-01), pages 1307 - 1319, XP002596752, ISSN: 1099-498X, DOI: 10.1002/JGM.967
- GRIMM D ET AL: "E Pluribus Unum: 50 Years of Research, Millions of Viruses, and One Goal-Tailored Acceleration of AAV Evolution", MOLECULAR THERAPY, vol. 23, no. 12, 1 December 2015 (2015-12-01), US, pages 1819 - 1831, XP055372394, ISSN: 1525-0016, DOI: 10.1038/mt.2015.173

## Description

The present invention relates to a process for producing a library of recombinant adeno-associated virus (AAV) particles which are encapsidated by a variety of different capsid polypeptides. Each recombinant AAV particle in the library comprises an AAV genome which comprises AAV ITRs flanking a first AAV cap gene encoding a first Cap polypeptide, wherein the particles in the library differ in the nucleotide sequences of their first AAV cap genes, and wherein each particle in the library is encapsidated by a Cap polypeptide which is encoded by the cap gene within its AAV genome. The library may be used to screen for recombinant AAV particles which have high specificity for cells of a target therapeutic tissue.

Adeno-associated viruses (AAVs) are single-stranded DNA viruses that belong to the *Parvoviridae* family. It is a non-pathogenic virus that generates only a limited immune response in most patients. The virus cellular and tissue tropism is defined by the capsid on the surface of the AAV particles. Some capsids allow infection of a broad range of host cells, including both dividing and non-dividing cells, whilst others are considerably more restricted. Some do not infect standard production or manufacturing cell lines such as HEK293 cells.

Over the last few years, vectors derived from AAVs have emerged as an extremely useful and promising mode of gene delivery. This is owing to the following properties of these vectors:
- AAVs are small, non-enveloped viruses and they have only two native genes (rep and *cap*). Thus, they can be easily manipulated to develop vectors for different gene therapies. This is achieved by the removal of the *rep* and *cap* genes in the AAV genome and replacing these sequences with exogenous sequences (transgenes) that may provide therapeutic benefit to a patient.
- AAV particles are not easily degraded by shear forces, enzymes or solvents. This facilitates easy purification and final formulation of these viral vectors.
- AAVs are non-pathogenic and have a low immunogenicity. The use of these vectors further reduces the risk of adverse inflammatory reactions. Unlike other viral vectors, such as lentivirus, herpes virus and adenovirus, AAVs are harmless and are not thought to be responsible for causing any human disease.
- Genetic sequences up to approximately 4500 bp can be delivered into a patient using AAV vectors.
- Whilst wild-type AAV vectors have been shown to sometimes insert genetic material into human chromosome 19, this property is generally eliminated from most AAV gene therapy vectors by removing rep and cap genes from the viral genome. In such cases, the virus remains in an episomal form within the host cells. These episomes remain intact in non-dividing cells, while in dividing cells they are lost during cell division.

The native AAV genome comprises two genes each encoding multiple open reading frames (ORFs): the rep gene encodes non-structural proteins that are required for the AAV life-cycle and site-specific integration of the viral genome; and the cap gene encodes the structural capsid proteins.

In addition, these two genes are flanked by inverted terminal repeat (ITR) sequences consisting of 145 bases that have the ability to form hairpin structures. These hairpin sequences are required for the primase-independent synthesis of a second DNA strand and the integration of the viral DNA into the host cell genome.

In order to eliminate any integrative capacity of the virus, some recombinant AAV *vectors remove rep and cap from the DNA of the viral genome. To produce such* vectors, the desired transgene(s), together with a promoter(s) to drive transcription of the transgene(s), is inserted between the inverted terminal repeats (ITRs); and the *rep* and *cap* genes are provided in *trans* from either a second plasmid or a helper virus encoding the *rep* and or *cap* genes. Helper genes such as adenovirus E4, E2a and VA genes are also provided by either a plasmid or a helper virus. *rep, cap* and helper genes may be provided on additional plasmids that are transfected into cells or via a helper virus.

Traditionally, the production of AAV vectors has been achieved through a number of different routes.

Initially, AAV was generated using wild-type (WT) Adenovirus serotype 5 whilst transfecting cells with plasmids encoding the rep and cap genes and the AAV genome. This allowed the WT adenovirus to provide a number of factors in *trans* that facilitated virus replication. However, there are a number of limitations to this approach: for example, each batch of AAV must be separated from the Adenoviral (AV) particles after manufacture to provide a pure product and ensuring that all Ad5 has been removed is challenging. Moreover, the fact that during production the cell is devoting considerable resources to the production of Adenoviral particles rather than AAV is also undesirable.

In other systems, stable packing cell lines expressing the rep and cap genes have been used. In such systems, the rep and cap genes are integrated into the cell genomes, hence obviating the need for plasmid-based rep and cap genes. However, these genes are usually only integrated at low frequency (e.g. 1-2 copies per cell) due to their inherent toxicity. These systems require the infection with adenoviral vectors.

More recently, the adenovirus-based systems have been replaced with plasmids encoding the sections of the Adenovirus genome required for AAV production.

However, owing to their restricted cell tropism, some cells of therapeutic value are difficult to transduce or infect using existing AAV's encapsulated by existing Cap polypeptides. Moreover, other cells are only currently able to be transduced or infected with broad host-range (i.e. non-specific) AAVs, which can lead to undesirable non-specific infection or transduction of patients' cells during clinical use.

There remains a need, therefore, for processes to produce a variety of AAVs having a range of abilities to infect or transduce new ranges of host cells or abilities to infect or transduce certain host cells with greater specificity.

The inventors have now developed a process whereby a DNA library of recombinant AAV genomes is introduced into first host cells, the members of the library having different first *cap* genes in their genomes but wherein the *cap* genes are not expressed. For example, a repressor may be expressed in the first host cells which represses or prevents expression of the first *cap* genes; or the first AAV *cap* genes may be operably-associated with an inducible promoter which is not present in the first host cells. A quantity of first AAV particles are produced in first host cells (e.g. a manufacturing cell line); each of these particles are encapsidated by common capsid polypeptides having a tropism towards second host cells. The first AAV particles are then used to infect or transduce second host cells under conditions such that the first *cap* genes are expressed and second AAV particles are produced, wherein the second AAV particles are each encapsidated by the (different) capsid polypeptides which are encoded within their own AAV genomes. In this way, a library of recombinant AAV particles is produced, the particles having a range of different capsids and hence different tropisms towards host cells and each AAV particle encodes within it the *cap* gene that encodes for the Cap polypeptides that are encapsulating said AAV particle. This thereby creates a phenotype to genotype link.

Virus display libraries have been produced previously, but the expression of the first AAV *cap* genes has not been actively prevented in these previous methods.

In WO2004/020600, the first host cells were insect cells; the expression of the first (wt) *cap* genes is silenced in such cells. This method was therefore limited to the use of such cells.

In Muller et al. (Nature Biotech. (2003) 21:9, 1040-1046), there was no attempt to prevent expression of the first cap genes in the first host cells, thus leading to the production of first AAV particles which were encapsidated by a mixture of wild-type and library *cap* genes. Due to homologous recombination between wild-type and library AAV genomes, some wild-type AAVs were produced which contaminated the final virus display library. This latter problem was mitigated to some extent in the method of Waterkamp et al. (J. Gene Med. (2006); 8, 1307-1319) by the use of a synthetic *cap* gene which was codon-optimised to reduce homologous recombination with the library AAV genomes.

The approach of the current invention seeks to use different capsids in the different stages such that the capsids are distinct from each other. The first capsid is suitable for infecting a packaging cell line, such as HEK293, whereas the second capsid is designed to infect the final target cells. This approach reduces the homology between the AAV capsids, thus reducing any homologous recombination issues.

It is an object of the invention therefore to provide a process for producing a library of recombinant AAV particles, the AAV particles being encapsidated by a variety of different capsids, thus conferring a variety of different tropisms towards host cells. The library may be used in screening for recombinant AAV particles which have a specificity or an enhanced specificity for defined host cells.

In one embodiment, the invention provides a process for producing a library of recombinant AAV particles, the process comprising the steps:
(a) introducing, into a population of first host cells, a DNA library comprising a plurality of DNA molecules, wherein each DNA molecule in the library comprises a recombinant AAV genome which comprises ITRs flanking a first AAV cap gene encoding first Cap polypeptides, and wherein DNA molecules in the library differ in the nucleotide sequences of their first AAV cap genes;
(b) culturing the population of first host cells under conditions such that:
   (i) a second AAV *cap* gene encoding second Cap polypeptides is expressed in the first host cells, wherein the second Cap polypeptides are ones which confer a tropism on AAV particles encapsidated by such polypeptides towards second host cells,
   (ii) an AAV *rep* gene and viral helper genes are expressed in the first host cells, and
   (iii) first AAV Cap polypeptides are not produced in the first host cells, by virtue of the expression of a repressor in the first host cells which represses or prevents expression of the first AAV Cap polypeptides or wherein the first AAV *cap* gene is operably-associated with an inducible promoter and the inducer is not present in the first host cells,

   wherein the AAV *rep* gene and second cap gene are both present in the genome of a recombinant adenovirus which is present in the first host cells,
   wherein first recombinant AAV particles are produced which are encapsidated by the second Cap polypeptides;
(c) infecting a population of second host cells with first recombinant AAV particles;
(d) culturing the second population of host cells in a culture medium under conditions such that
   (i) first AAV Cap polypeptides are produced, and
   (ii) an AAV *rep* gene and viral helper genes are expressed in the second host cells,

   wherein, if the first AAV cap gene is operably-associated with an inducible promoter, the inducer is present in the second population of host cells,
   wherein the AAV *rep* gene is present in the genome of a recombinant adenovirus which is present in the second population of host cells,
   wherein an AAV particle library of second recombinant AAV particles is produced, wherein each second recombinant AAV particle in the library comprises an AAV genome which comprises ITRs flanking a first AAV *cap* gene encoding a first Cap polypeptide, wherein the particles in the library differ in the nucleotide sequences of their first AAV *cap* genes, and wherein each particle in the library is encapsidated by Cap polypeptides which are encoded by the (first) *cap* gene in its AAV genome;
   and optionally
(e) purifying and/or isolating a library of second recombinant AAV particles from the second host cells or from the culture medium.

Preferably, the first *cap* genes are operably-associated with a regulatory element (e.g. a repressor element or inducible promoter) such that the first Cap polypeptides are not produced in the first host cells.

More preferably, the first cap genes are operably-associated with a regulatory element (e.g. a repressor element or inducible promoter) such that the first cap genes are not expressed in the first host cells.

### DETAILS OF SEQUENCES

The following sequences are given in the Sequence Listing, which forms part of the description of this patent application.

| **SEQ ID NO:** | **Description** | **Form** |
|---|---|---|
| 1 | AAV1 capsid | Nucleotide |
| 2 | AAV1 capsid | Amino acid |
| 3 | AAV2 capsid | Nucleotide |
| 4 | AAV2 capsid | Amino acid |
| 5 | AAV3 capsid | Nucleotide |
| 6 | AAV3 capsid | Amino acid |
| 7 | AAV4 capsid | Nucleotide |
| 8 | AAV4 capsid | Amino acid |
| 9 | AAV5 capsid | Nucleotide |
| 10 | AAV5 capsid | Amino acid |
| 11 | AAV6 capsid | Nucleotide |
| 12 | AAV6 capsid | Amino acid |
| 13 | AAV7 capsid | Nucleotide |
| 14 | AAV7 capsid | Amino acid |
| 15 | AAV8 capsid | Nucleotide |
| 16 | AAV8 capsid | Amino acid |
| 17 | AAV9 capsid | Nucleotide |
| 18 | AAV9 capsid | Amino acid |
| 19 | AAV2 *rep gene* | Nucleotide |
| 20 | AAV2 Rep78 | Nucleotide |
| 21 | AAV2 Rep78 | Amino acid |
| 22 | AAV2 Rep68 | Nucleotide |
| 23 | AAV2 Rep68 | Amino acid |
| 24 | AAV2 Rep52 | Nucleotide |
| 25 | AAV2 Rep52 | Amino acid |
| 26 | AAV2 Rep40 | Nucleotide |
| 27 | AAV2 Rep40 | Amino acid |
| 28 | TetR binding site | Nucleotide |
| 29 | Modified AV Major Late Promoter | Nucleotide |
| 30 | Modified AV Major Late Promoter | Nucleotide |
| 31 | 4C5G binding site (synthetic) | Nucleotide |
| 32 | CMV promoter containing two 4C5G binding sites (synthetic) | Nucleotide |
| 33 | TetR-V36A-E37A-P39K coding sequence (synthetic) | Nucleotide |
| 34 | Cumate binding site (Pseudomonas putida) | Nucleotide |
| 35 | Minimal CMV promoter containing Cumate activator binding sites (synthetic) | Nucleotide |
| 36 | Cumate activator (CmyR-VP16) protein coding sequence (synthetic) | Nucleotide |

In Step (a), a DNA library is introduced into a population of first host cells.

As used herein, the term "introducing" includes transformation, and any form of electroporation, conjugation, infection, transduction or transfection, *inter alia.* The term "introduced" is similarly interpreted, *mutatis mutandis.* This introduction could, for example, be by transfection of plasmid, linear, or transposon DNA encoding the recombinant AAV genomes, or by infection with a viral vector encoding the AAV genomes, e.g. using an RNA virus such as a retrovirus that will introduce the recombinant AAV genomes into the first host cell genome.

Preferably, the DNA library is in the form of a library of plasmids or vectors, transposons, linear DNA molecules or lentiviral particles or lentiviral vectors.

As used herein, the term "nucleic acid" refers to DNA and RNA molecules, preferably DNA molecules.

DNA transposons are natural and easily-controllable DNA delivery vehicles that can be used as tools for versatile gene delivery and gene discovery applications ranging from transgenesis to functional genomics and gene therapy. Transposons are simply organized: they encode a transposase protein in their simple genome flanked by inverted terminal repeats (ITRs) that carry transposase binding sites necessary for transposition. Transposons move through a "cut-and-paste" mechanism that involves excision of the element from the DNA and subsequent integration of the element into a new sequence environment.

DNA transposons are classified into different families depending on their sequence, Inverted Terminal Repeats, and/or target site duplications. The families in Subclass I are: Tc1/mariner, PIF/Harbinger, hAT, Mutator, Merlin, Transib, P, *piggyBac,* CACTA, and *Sleeping Beauty.* Helitron and Maverick transposons belong to Subclass II, since they are replicated and do not perform double-strand DNA breaks during their insertion. In some embodiments, the transposon is based upon a Class II, Subclass I transposon. In other embodiments, the transposon is based upon a *piggyBac, Sleeping Beauty* or a Tol2 transposon, or a variant or derivative thereof.

Retroviruses (including lentiviruses) are positive-sense RNA viruses that undergo a complex life cycle involving the reverse transcription of their genome into DNA, which subsequently becomes integrated into the host cell genome following viral infection.

They are capable of inserting their genomes, as DNA, into almost any loci in the genome of target cells and mediating long-term expression of virus genes, with the DNA being copied into each daughter cell when the infected cell divides.

Although wild-type retro/lentiviruses can replicate in host cells, retro/lentivirus vectors are typically disabled in a range of ways to remove their ability to replicate and cause disease. The production and use of lentiviral vectors is described in WO2019/058108.

The aim of Steps (a)-(b) is to produce a quantity of first recombinant AAV particles for use in Steps (c) and (d). Hence the first host cells are preferably ones which are readily capable of having DNA molecules comprising recombinant AAV genomes introduced into them and of producing first recombinant AAV particles.

Preferably, the first host cells are from an AAV production cell line or an AAV manufacturing cell line, i.e. a cell line which comprises all of the polypeptides which are necessary for AAV capsid production and AAV maturation. The first host cells may comprise one or more different types of cells (e.g. a mixture of HEK293 and PerC6 cells). Preferably, the first host cells are all of the same type.

The first host cell is preferably a mammalian cell. Examples of mammalian cells include those from any organ or tissue from humans, mice, rats, hamsters, monkeys, rabbits, donkeys, horses, sheep, cows and apes. Preferably, the cells are human cells. The cells may be primary or immortalised cells.

Preferred first host cells include HEK-293, HEK 293T, HEK-293E, HEK-293 FT, HEK-293S, HEK-293SG, HEK-293 FTM, HEK-293SGGD, HEK-293A, MDCK, C127, A549, HeLa, CHO, mouse myeloma, PerC6, 911 and Vero cell lines. HEK-293 cells have been modified to contain the E1A and E1B proteins and this obviates the need for these proteins to be supplied on a Helper Plasmid. Similarly, PerC6 and 911 cells contain a similar modification and can also be used. Most preferably, the human cells are HEK293, HEK293T, HEK293A, PerC6, 911 or HeLaRC32. Other preferred cells include Hela cells.

The DNA library comprises a plurality of DNA molecules, wherein each molecule in the library comprises a recombinant AAV genome which comprises ITRs flanking a first AAV *cap* gene encoding first Cap polypeptides, and wherein DNA molecules in the library differ in the nucleotide sequences of their first AAV cap genes. In certain embodiments, the plasmid library may comprise at least 10⁴, 10⁵, 10⁶, 10⁷, 10⁸, 10⁹ or more different first AAV cap genes.

The DNA library can be produced via a range of approaches. New developments in DNA synthesis technology now allow the precise controlled synthesis of large DNA libraries that have been rationally designed *in silico.* These libraries can be synthesised and cloned into a range of vectors that are suitable for the delivery of the DNA library into first host cells, such as a transposon or retroviral vector plasmid, or even transfected as a linear DNA library if the library has been synthesised with the flanking AAV ITRs and appropriate control elements for the *cap* gene.

Alternatively, specific regions of the *cap* gene can be mutated using degenerate primers that, via PCR, will produce a range of DNA products having a number of mutations in the amino acids that are hypothesised to endow new tropisms upon the AAV particles. When designing such degenerate libraries, care must be taken to avoid the introduction of stop codons where possible, or amino acids that are known to be non-functional on the AAV particle surface. However, the nature of degenerate oligo synthesis means that this is not always completely achievable. To improve the quality of the degenerate oligonucleotide pool, controlled synthesis on a silicon surface can be used to ensure that only the desired sequences are synthesised.

DNA libraries of AAV capsids can be rationally designed, e.g. as follows:
1. AAV capsid amino acid sequences for Adeno-associated virus (AAV) may be obtained from NCBI, Prosite. These sequences can then be compared by performing multiple-sequence alignment (MSA) and constructing Neighbour-joining phylogeny with known type sequences from RefSeq to identify capsid sequences belonging to specific serotypes.
2. From the MSA used to construct the phylogeny, regions of highly-conserved residues can be distinguished from those that are variable between the capsids of interest. Variable regions within the capsid are more likely to be less essential and are open to evolutionary forces that may direct the evolution of a trait, for example tropism. This is in contrast to the more conserved regions that are essential and may be lethal if mutations were to occur in them.
3. Within the identified variable sites, specific residues may be identifiable that provide one AAV serotype with a specific tropism (sites of interest), over other AAV capsids that do not have this residue present. These sites of interest can then be compared to the 3D X-ray crystallography (XRC) structure of an AAV6 capsid allowing the assessing of their spatial and structural properties.
4. Several filters can then be applied to reduce the number of variants, but also to make the selection of variants more informed. Four criteria can be chosen to inform the selection process:
   a. Firstly, remove glycine and proline residues from sites of interest that are known to have abnormal bond angles and thus could have a significant impact on capsid integrity, from the sites of interest.
   b. Secondly, measure if the selected residues are present on the surface and present in a conformation which could facilitate chemical interactions e.g. by calculating how accessible a residue is to solvents. Residues that are accessible to solvents, and thus are capable of interacting with tissue/ cell receptors can be shortlisted.
   c. Thirdly, identify those residues known from previous studies to have a direct role in tropism and specifically those driven by receptor-based interactions.
   d. Finally, determine if residues are exposed on the surface of the capsid or buried within the capsid and thus inaccessible to tropic interactions.
5. Lastly, in order to construct a library of size 10^7, combinations of amino acid variants at different positions can be selected that would result in the required number of variants in the library.

Alternatively, if a truly random library is required, this can be achieved by using a mutagenic PCR. This typically involves a PCR reaction of the region of DNA that is to be mutated, in this case the AAV cap gene, in the presence of increasing concentrations of magnesium. This is known by those in the art to increase the error rate of many polymerases. In this approach it is also preferable to use a non-proof-reading polymerase such as Taq.

Adeno-associated viruses (AAV) are small (approx. 20 nm) replication-defective, non-enveloped viruses. In some embodiments, the AAV is an Adeno-associated dependoparvovirus A. In other embodiments, the AAV is an Adeno-associated dependoparvovirus B.

AAV particles are formed from capsid proteins which encapsidate the ssDNA AAV genome.

The wild-type AAV genome comprises two genes each encoding multiple open reading frames (ORFs): the rep gene encodes non-structural proteins that are required for the AAV life-cycle and site-specific integration of the viral genome; and the cap gene encodes the structural capsid proteins.

As used herein, the term "recombinant AAV particle" refers to an AAV particle which comprises a recombinant AAV genome. As used herein, the term "recombinant AAV genome" refers to an AAV genome comprising AAV inverted terminal repeats (ITRs) flanking an intervening sequence, preferably wherein the intervening sequence is more than 100bp. The intervening sequence comprises a first AAV cap gene, encoding first Cap (capsid) polypeptides. The recombinant AAV genome preferably does not comprise an AAV rep gene. The recombinant AAV genome may comprise a transgene (e.g. a reporter gene), within the flanking ITRs.

Preferably, the term "recombinant AAV genome" refers to an AAV genome comprising a first AAV cap gene flanked by AAV inverted terminal repeats (ITRs).

As used herein, the terms "AAV genome", "AAV Transfer vector" and "Transfer Plasmid" are used interchangeably. They all refer to a vector comprising 5'- and 3'-viral (preferably AAV) inverted terminal repeats (ITRs) flanking an intervening sequence.

The transgene may be a coding or non-coding sequence. It may be genomic DNA or cDNA. Preferably, the transgene encodes a polypeptide or a fragment thereof. Preferably, the transgene is operably-associated with one or more transcriptional and/or translational control elements (e.g. an enhancer, promoter, terminator sequence, etc.).

In a preferred embodiment of the invention, the transgene encodes a reporter gene. The reporter gene preferably produces a colorimetric, fluorescent, or luminescent signal that will allow cells that contain and express the reporter gene to be identified from surrounding cells that do not contain the reporter gene.

In some preferred embodiments, the reporter gene is one which produces a fluorescent signal. Preferred examples include genes encoding enhanced green fluorescent protein (EGFP), MCherry, and DsRed. Many other derivates or alternative equivalents are available of such proteins and are well known to those in the art.

In some embodiments, the reporter gene is one which produces a colorimetric signal. Preferred examples include genes encoding secreted alkaline phosphatase (SEAP) or Beta Galactosidase (beta gal).

In some embodiments, the reporter gene is one which produces a luminescent signal. Preferred examples include genes encoding *Photinus pyralis* (firefly) luciferase and *Renilla reniformis* (sea pansy) luciferase. Many other luciferases are now available and are well known to those in the art such as those from copepods and dinoflagellates.

In some embodiments, the transgene codes for a therapeutic polypeptide or a fragment thereof. Examples of preferred therapeutic polypeptides include antibodies, CAR-T molecules, scFV, BiTEs, DARPins, GPCRs, T-cell receptors and human genes. In some embodiments, the therapeutic polypeptide is a functioning copy of a gene involved in human blood production or is a blood component, e.g. Factor IX, or those involved in beta and alpha thalassemia or sickle cell anaemia. In some embodiments, the therapeutic polypeptide is a functioning copy of a gene involved in immune function such as that in severe combined immune-deficiency (SCID) or Adenosine deaminase deficiency (ADA-SCID). In some embodiments, the therapeutic polypeptide is a protein which increases/decreases proliferation of cells, e.g. a growth factor receptor. In some preferred embodiments, the transgene encodes a CRISPR enzyme (e.g. Cas9, dCas9, Cpf1 or a variant or derivative thereof) or a CRISPR sgRNA.

The plasmids in the library each comprise a first AAV cap gene. As used herein, the term "cap gene" refers to a gene that encodes one or more open reading frames (ORFs), wherein each of said ORFs encodes an AAV Cap structural protein, or variant or derivative thereof. These AAV Cap structural proteins (or variants or derivatives thereof) form the AAV capsid.

The three Cap proteins are VP1, VP2 and VP3, which are generally 87kDa, 72kDa and 62kDa in size, respectively. Hence the *cap* gene is one which encodes the three Cap proteins VP1, VP2 and VP3. In the wild-type AAV, these three proteins are translated from the p40 promoter to form a single mRNA. After this mRNA is synthesized, either a long or a short intron can be excised, resulting in the formation of a 2.3 kb or a 2.6 kb mRNA. The AAV capsid is composed of 60 capsid protein subunits (VP1, VP2, and VP3) that are arranged in an icosahedral symmetry in a ratio of 1:1:10, with an estimated size of 3.9 MDa.

As used herein, the term "*cap* gene" includes wild-type *cap* genes and derivatives thereof, and artificial *cap* genes which have equivalent functions.

The AAV *cap* gene sequences and Cap polypeptide sequences for AAV serotypes 1-9 are given herein in SEQ ID NOs: 1-18, respectively.

As used herein, the term "*cap* gene" (including first or second *cap* gene) or Cap polypeptide-encoding sequence preferably includes, but is not limited to:
(a) a polynucleotide molecule whose nucleotide sequence comprises or consists of the nucleotide sequence given in any one of SEQ ID NOs: 1, 3, 5, 7, 9, 11, 13, 15 or 17 (preferably SEQ ID NO: 17);
(b) a polynucleotide molecule whose nucleotide sequence comprises or consists of a variant of the nucleotide sequence given in any one of SEQ ID NOs: 1, 3, 5, 7, 9, 11, 13, 15 or 17 (preferably SEQ ID NO: 17), the variant having at least 70%, 80%, 85%, 90%, 95% or 99% (preferably at least 95%) sequence identity to any one of SEQ ID NOs: 1, 3, 5, 7, 9, 11, 13, 15 or 17 (preferably SEQ ID NO: 17); and
(c) a polynucleotide molecule whose nucleotide sequence comprises or consists of a nucleotide sequence which encodes:
   (i) a polypeptide whose amino acid sequence is given in any one of SEQ ID NOs: 2, 4, 6, 8, 10, 12, 14, 16, or 18 (preferably SEQ ID NO: 18), or
   (ii) a variant of (i), the variant having at least 70%, 80%, 85%, 90%, 95% or 99% (preferably at least 95%) sequence identity to any one of SEQ ID NOs: 2, 4, 6, 8, 10, 12, 14, 16, or 18 (preferably SEQ ID NO: 18).

Preferably, the variant is or encodes one or more VP1, VP2 and VP3 polypeptides.

Preferably, the Cap polypeptide-encoding sequence is a polypeptide whose amino acid sequence is given in any one of SEQ ID NOs: 2, 4, 6, 8, 10, 12, 14, 16, or 18, or (ii) a variant of (i), the variant having at least 70%, 80%, 85%, 90%, 95% or 99% (preferably at least 95%) sequence identity to any one of SEQ ID NOs: 2, 4, 6, 8, 10, 12, 14, 16, or 18.

In some embodiments, as used herein, the term "first cap gene" or first Cap polypeptide-encoding sequence preferably includes, but is not limited to:
(a) a polynucleotide molecule whose nucleotide sequence comprises or consists of the nucleotide sequence given in SEQ ID NO: 17:
(b) a polynucleotide molecule whose nucleotide sequence comprises or consists of a variant of the nucleotide sequence given in SEQ ID NO: 17, the variant having at least 70% or 80% (preferably at least 80%) sequence identity to SEQ ID NO: 17, and
(c) a polynucleotide molecule whose nucleotide sequence comprises or consists of a nucleotide sequence which encodes:
   (i) a polypeptide whose amino acid sequence is given in SEQ ID NO: 18 or
   (ii) a variant of (i), the variant having at least 70% or 80% (preferably at least 80%) sequence identity to SEQ ID NO: 18.

More preferably, the first Cap polypeptide-encoding sequence is a polypeptide whose amino acid sequence is given in SEQ ID NO: 18, or a variant thereof, the variant having at least 70% or 80% (preferably at least 80%) sequence identity to SEQ ID NO: 18.

In some embodiments, as used herein, the term "second *cap* gene" or second Cap polypeptide-encoding sequence preferably includes, but is not limited to:
(a) a polynucleotide molecule whose nucleotide sequence comprises or consists of the nucleotide sequence given in any one of SEQ ID NOs: 1, 3, 5 or 11 (preferably SEQ ID NO: 3);
(b) a polynucleotide molecule whose nucleotide sequence comprises or consists of a variant of the nucleotide sequence given in any one of SEQ ID NOs: 1, 3, 5 or 11, (preferably SEQ ID NO: 3), the variant having at least 70% or 80% (preferably at least 80%) sequence identity to any one of SEQ ID NOs: 1, 3, 5 or 11 (preferably SEQ ID NO: 3); and
(c) a polynucleotide molecule whose nucleotide sequence comprises or consists of a nucleotide sequence which encodes:
   (i) a polypeptide whose amino acid sequence is given in any one of SEQ ID NOs: 2, 4, 6 or 12 (preferably SEQ ID NO: 4), or
   (ii) a variant of (i), the variant having at least 70% or 80% (preferably at least 80%) sequence identity to any one of SEQ ID NOs: 2, 4, 6 or 12 (preferably SEQ ID NO: 4).

More preferably, the second Cap polypeptide-encoding sequence is a polypeptide whose amino acid sequence is given in any one of SEQ ID NOs: 2, 4, 6 or 12, or a variant thereof, the variant having at least 70% or 80% (preferably at least 80%) sequence identity to any one of SEQ ID NOs: 2, 4, 6 or 12.

The DNA molecules in the library differ in the nucleotide sequences of their first AAV cap genes.

In the context of this invention, it is not essential that all of the first AAV cap genes encode functional capsid polypeptides. For example, random mutations of the AAV cap genes may result in the production of a mixture of functional and non-functional capsid polypeptides. The use of any such mutation-producing processes should be limited, however, such that some functional capsid polypeptides are capable of being produced by at least some members of the library.

Preferably, the first AAV cap gene will be a cap gene or first cap gene as defined above. Preferably, the Cap polypeptide-encoding sequence will be as defined above.

The first AAV Cap polypeptides are not produced in the first host cells. The second AAV Cap polypeptides preferably not produced in the second host cells.

Although AAV genomes comprising first AAV cap genes are present in the first host cells, in the process of the invention, first AAV Cap polypeptides are not produced in the first host cells. This prevents the encapsidation of the first AAV recombinant particles by capsid polypeptides which are encoded by the particles' endogenous first cap genes.

The expression of the second AAV *cap* gene in the first host cells ensures that the first recombinant AAV particles which are produced by the first host cells are all encapsidated by second AAV Cap polypeptides.

The first AAV Cap polypeptides may be prevented from being produced by any suitable means. In some embodiments, a repressor is expressed in the first host cells which represses or prevents expression of the first AAV Cap polypeptides. The repressor is not expressed in the second host cells. The repressor may be one which represses or prevents transcription of the first AAV cap gene, which represses or prevents translation of the first AAV cap mRNA or which represses or prevents production or function of one or more AAV Cap polypeptides.

For example, the expression of the first AAV cap gene may be placed under the control of a promoter which comprises a repressor-binding site; and a repressor which binds to the repressor-binding site is expressed in the first host cells.

The repressor may, for example, be expressed endogenously within the first host cells or it may have been introduced into the first host cells (e.g. a plasmid or vector encoding an antisense RNA or a repressor polypeptide).

In some embodiments, the repressor is an anti-cap antisense RNA, wherein the antisense RNA binds to mRNA from the first cap gene but not to mRNA from the second cap gene.

Preferably, the antisense RNA is capable of binding to and inhibiting translation of a first AAV Cap mRNA (compared to the translation of a control AAV Cap mRNA in the absence of the antisense RNA).

In some embodiments, the antisense RNA is capable of binding to and increasing the degradation rate of a first AAV Cap mRNA (compared to the degradation rate of a control AAV Cap mRNA in the absence of the antisense RNA).

The antisense RNA is a single-stranded RNA molecule. Preferably, the antisense RNA is a short hairpin RNA (shRNA) that is processed to produce a siRNA or a mature microRNA (miRNA).

In some embodiments, the antisense RNA binds to a first AAV Cap mRNA which encodes VP1. In some embodiments, the antisense RNA binds to a first AAV Cap mRNA which encodes VP2. In some embodiments, the antisense RNA binds to a first AAV Cap mRNA which encodes VP3.

In some embodiments, the antisense RNA binds to a region of a first AAV Cap mRNA which is common to all mRNA which encode VP1, VP2 or VP3 (of a particular strain of AAV).

In other embodiments, the antisense RNA binds to the 5' or 3' UTR of a first AAV Cap mRNA, preferably to the 3' UTR. An untranslated region (or UTR) refers to either of two sections, one on each side of a coding sequence on a strand of mRNA. If it is found on the 5' side, it is called the 5' UTR (or leader sequence), or if it is found on the 3' side, it is called the 3' UTR (or trailer sequence). The 3' UTR is found following the translation stop codon. The 3' UTR plays a role in translation termination as well as post-transcriptional modification.

The degree of complementary nucleotide sequence identity between the antisense RNA (i.e. the anti-Cap mRNA antisense RNA) and the corresponding region of first Cap mRNA is preferably at least 70%, 80%, 90%, 95% or 100%, preferably 100%.

The length of antisense RNA which has complementary sequence identity to the first Cap mRNA is preferably 18-27 nucleotides, more preferably 20-22 nucleotides, and most preferably 21 nucleotides.

Preferably, the antisense RNA inhibits the translation of the first Cap mRNA by at least 70%, preferably at least 80% or at least 90%. The degree of inhibition of the Cap mRNA may be assayed by Western blot, using an anti-Cap antibody.

Preferably, the antisense RNA increases the degradation rate of the first Cap mRNA and thereby reduces translation and/or expression of the first Cap polypeptide. The degree of first Cap mRNA degradation is preferably at least 70%. mRNA degradation may be assayed by RT-QPCR against the Cap mRNA or Northern blotting against the same target.

In some embodiments, it is preferable that the antisense RNA increases the accumulation of the first Cap mRNA in processing bodies. This can be determined by staining for the presence of first Cap mRNA in fixed cells by microscopy using a labelled RNA that can hybridise to the Cap mRNA sequences. An shRNA that matures into an siRNA that can bind to the conserved regions of the first Cap mRNA (e.g. the 3' UTR) could be inserted into the genome of the first host cells but not the second host cells. This would allow the selective degradation of the first Cap mRNAs in the first host cells.

miRNAs (microRNAs) are short regulatory RNAs that function by guiding the miRNA-induced silencing complex (miRISC) to RNA targets bearing a complementary "seed" sequence. The binding of an miRNA to the seed sequence results in attenuation of gene expression (protein production for coding genes); possible mechanisms include translational inhibition and target mRNA destabilization and decay. For example, the first Cap mRNA sequence could be modified to insert one or more binding sites for a microRNA which is produced in first host cells but not in second host cells. This would result in the degradation of the first Cap mRNA in the first host cells.

In other embodiments, the first AAV cap gene is operably-associated with an inducible promoter and the inducer is not present in the first host cells. The inducer is present in the second host cells.

In other embodiments, the first AAV cap gene is operably-associated with a repressible promoter and the repressor is present in the first host cells. The repressor is not present in the second host cells.

A second AAV cap gene encoding second Cap polypeptides is expressed in the first host cells. This ensures that the first recombinant AAV particles which are produced in the first host cells are all encapsidated by second AAV Cap polypeptides.

The second AAV cap gene may, for example, be expressed endogenously within the first host cells or it may be or have been introduced into the first host cells, e.g. a plasmid, vector, or adenoviral vector encoding the second AAV cap gene.

The first recombinant AAV particles are encapsidated by capsids comprising second AAV capsid polypeptides which confer a tropism towards the second host cells.

The second host cells are therefore ones which are capable of being infected by AAV particles which are encapsidated by second AAV capsid polypeptides.

Some of the second recombinant AAV particles will be encapsidated by capsids comprising first AAV capsid polypeptides which confer a tropism towards third host cells.

The third host cells are ones which are capable of being infected by AAV particles which are encapsidated by first AAV capsid polypeptides.

AAV serotypes are determined by the AAV host cell tropisms. A serotype is a distinct variation within a species of virus. These viruses are classified together based on their cell surface (i.e. capsid) antigens, allowing the epidemiologic classification of viruses to the subspecies level.

AAV capsid proteins contain 12 hypervariable surface regions. The naturally-occurring capsid polypeptides vary from each other in their amino acid sequences. The primary regions of variation are recognised as being within the VP3 region of the capsid genes. However, the VP3 region is shared with the VP1 and VP2 coding sequences. Therefore, it is more accurate to say that the variable regions of the Cap polypeptides are more commonly found in the C-terminal half of the cap gene and, as such, the variation is generally shared by all of the capsid coding sequences. Preferably, therefore, the first and second recombinant AAV particles are of different serotypes.

11 different AAV serotypes are known. All of the known serotypes can infect cells from multiple diverse host cell types. The serotypes of the first and second recombinant AAV particles may be selected from the group consisting of serotypes 1, 2, 3, 4, 5, 6, 7, 8, 9, 10 and 11. In some embodiments, the serotypes of the first and second recombinant AAV particles are selected from the group consisting of serotypes 1, 2, 5, 6, 7, 8 or 9. Preferably, the first recombinant AAV particles are of serotype 1, 2, 3, or 6 (i.e. AAV1, AAV2, AAV3 or AAV6).

The second recombinant AAV particles will be encapsidated by Cap polypeptides which are encoded by first cap genes from the DNA library. The second recombinant AAV particles might not, therefore, confer a known or established serotype on those second recombinant AAV particles: the second recombinant AAV particles might be encapsidated by Cap polypeptides which are derived from Cap polypeptides which confer a known or established serotype.

In some embodiments, the second recombinant AAV particles are of serotype 9 (i.e. AAV9) or are encapsidated by Cap polypeptides which are derived from Cap polypeptides which confer serotype 9 on AAV particles encapsidated by such polypeptides. Even more preferably, the second recombinant AAV particles are of a serotype, or modified or mutated derivative of a serotype, that has an increased tropism for the third host cells (target cells) compared to the tropism of AAV1-9.

Preferably, the nucleotide sequences of the first AAV cap genes are different from the nucleotide sequence of the second AAV cap gene. However, due to the size and nature of the DNA library, it is possible that the nucleotide sequence of the second AAV *rep* gene is present in the DNA library.

An AAV *rep* gene is expressed in the first and second host cells. The AAV *rep* gene which is expressed in the first host cells may have the same or different nucleotide sequence as the AAV rep gene which is expressed in the second host cells.

As used herein, the term "rep gene" refers to a gene that encodes one or more open reading frames (ORFs), wherein each of said ORFs encodes an AAV Rep non-structural protein, or variant or derivative thereof. These AAV Rep non-structural proteins (or variants or derivatives thereof) are involved in AAV genome replication and/or AAV genome packaging.

The wild-type rep gene comprises three promoters: p5, p19 and p40. Two overlapping messenger ribonucleic acids (mRNAs) of different lengths can be produced from p5 and from p19. Each of these mRNAs contains an intron which can be either spliced out or not using a single splice donor site and two different splice acceptor sites. Thus, six different mRNAs can be formed, of which only four are functional. The two mRNAs that fail to remove the intron (one transcribed from p5 and one from p19) read through to a shared terminator sequence and encode Rep78 and Rep52, respectively. Removal of the intron and use of the 5'-most splice acceptor site does not result in production of any functional Rep protein - it cannot produce the correct Rep68 or Rep40 proteins as the frame of the remainder of the sequence is shifted, and it will also not produce the correct C-terminus of Rep78 or Rep52 because their terminator is spliced out. Conversely, removal of the intron and use of the 3' splice acceptor will include the correct C-terminus for Rep68 and Rep40, whilst splicing out the terminator of Rep78 and Rep52. Hence the only functional splicing either avoids splicing out the intron altogether (producing Rep78 and Rep52) or uses the 3' splice acceptor (to produce Rep68 and Rep40). Consequently, four different functional Rep proteins with overlapping sequences can be synthesized from these promoters.

In the wild-type *rep* gene, the p40 promoter is located at the 3' end. Transcription of the Cap proteins (VP1, VP2 and VP3) is initiated from this promoter in the wild-type AAV genome.

The four wild-type Rep proteins are Rep78, Rep68, Rep52 and Rep40. Hence the wild-type *rep* gene is one which encodes the four Rep proteins Rep78, Rep68, Rep52 and Rep40.

As used herein, the term "*rep* gene" includes wild-type *rep* genes and derivatives thereof; and artificial *rep* genes which have equivalent functions. The wild-type *rep* gene encodes Rep78, Rep68, Rep52 and Rep40 polypeptides.

The full wild-type AAV (serotype 2) *rep* gene nucleotide sequence is given in SEQ ID NO: 19. The wild-type AAV (serotype 2) Rep78, Rep68, Rep52 and Rep40 nucleotide sequences are given herein in SEQ ID NOs: 20, 22, 24 and 26, respectively. The wild-type AAV (serotype 2) Rep78, Rep68, Rep52 and Rep40 amino sequences are given herein in SEQ ID NOs: 21, 23, 25 and 27, respectively.

As used herein, the term "rep gene" or Rep polypeptide-encoding sequence preferably includes, but is not limited to:
(a) a polynucleotide molecule whose nucleotide sequence comprises or consists of the nucleotide sequence given in any one of SEQ ID NOs: 19, 20, 22, 24 or 26 (preferably, SEQ ID NO: 19); and
(b) a polynucleotide molecule whose nucleotide sequence comprises or consists of a variant of the nucleotide sequence given in any one of SEQ ID NOs: 19, 20, 22, 24 or 26 (preferably SEQ ID NO: 19), the variant having at least 80%, 85%, 90%, 95% or 99% (preferably at least 95%) sequence identity to any one of SEQ ID NOs: 19, 20, 22, 24, or 26 (preferably SEQ ID NO: 19); and
(c) a polynucleotide molecule whose nucleotide sequence comprises or consists of a nucleotide sequence which encodes:
   (i) a polypeptide whose amino acid sequence is given in any one of SEQ ID NOs: 21, 23, 25 or 27, (preferably SEQ ID NO: 21), or
   (ii) a variant of (i), the variant having at least 80%, 85%, 90%, 95% or 99% (preferably at least 95%) sequence identity to any one of SEQ ID NOs: 21, 23, 25 or 27 (preferably SEQ ID NO: 21).

Preferably, the variant is or encodes one or more Rep78, Rep68, Rep52 and Rep40 polypeptides.

The AAV rep gene may be expressed endogenously within the first and/or second host cells or it may be or have been introduced into the first and/or second host cells, e.g. a plasmid or vector encoding the AAV rep gene, or in an adenoviral vector.

The rep and cap genes (and each of the protein-encoding ORFs therein) may be from one or more different viruses. For example, the rep gene may be from AAV2, whilst a cap gene may be from AAV5.

It is recognised by those in the art that the rep and cap genes of AAV vary by clade and isolate. The sequences of these genes from all such clades and isolates are encompassed herein.

Nucleic acid molecules encoding viral helper genes are also expressed in the first and second host cells. Helper genes are required to aid the replication of the AAV genome and the production of infectious AAV particles. Suitable viral helper genes include E1A , E1B, E4, and VA RNA, and optionally an E2A gene. The helper genes are viral helper genes, preferably from adenovirus, herpesvirus or poxvirus. Most preferably, the helper genes are adenovirus helper genes. Some host cells (e.g. HEK293 cells) have the adenoviral E1A and E1B genes stably integrated into their genomes and hence it is not necessary to introduce further copies of these latter genes into such host cells.

In order to produce recombinant AAV particles comprising the recombinant AAV genome, the recombinant AAV genome must be introduced into the host cells, and Rep and Cap polypeptides must be produced within the host cells or subsequently provided. Additionally, sufficient viral helper genes (e.g. E4, E1A, E1B and VA RNA, and optionally an E2A gene) are needed.

As used herein, the term "introducing" includes transformation, and any form of electroporation, conjugation, infection, transduction or transfection, *inter alia.* The term "introduced" is similarly interpreted, *mutatis mutandis.*

Nucleic acid molecules encoding the second AAV cap gene, the rep gene and the viral helper genes may independently be present in the first host cells or independently subsequently introduced into the first host cells in one or more of the following forms:
(i) stably integrated into the first host cell's genome;
(ii) present episomally within the first host cell;
(iii) present in a recombinant adenovirus in the first host cell;
(iv) introduced into the first host cell in a recombinant adenovirus; or
(v) introduced into the first host cell in a vector or plasmid.

Nucleic acid molecules encoding the rep gene and the viral helper genes may independently be present in the second host cells or independently subsequently introduced into the second host cells in one or more of the following forms:
(i) stably integrated into the second host cell's genome;
(ii) present episomally within the second host cell;
(iii) present in a recombinant adenovirus in the second host cell;
(iv) introduced into the second host cell in a recombinant adenovirus; or
(v) introduced into the second host cell in a vector or plasmid.

The forms of the nucleic acid molecules encoding the AAV rep genes and the viral helper genes in the first and second host cells may be the same or different.

In Step (a), in embodiments wherein the above (iv) or (v) are introduced into the host cells, this introduction may be before or after introduction of the plasmid library.

Preferably, a nucleic acid molecule encoding the second AAV cap gene is present in the first host cells or is introduced into the first host cells in the form of a vector or a plasmid, or in a recombinant adenovirus.

Preferably, the vector, plasmid or recombinant adenovirus is introduced into the first host cells either before or after or simultaneously with the introduction of the DNA library.

Most preferably, a nucleic acid molecule encoding the second AAV cap gene is introduced into the first host cells in the form of a plasmid or in the genome of a recombinant AV (e.g. such as that described in WO2019/020922 and further herein).

Preferably, a nucleic acid molecule encoding the AAV rep gene is introduced into the first host cells in the form of a plasmid or in the genome of a recombinant AV (e.g. such as that described in WO2019/020922 and further herein).

Preferably, a nucleic acid molecule encoding the AAV rep gene is introduced into the second host cells in the genome of a recombinant AV (e.g. such as that described in WO2019/020922 and further herein).

Preferably, nucleic acid molecules encoding the viral helper genes are present in the first and/or second host cells or are introduced into the first and/or host cells in the form of a helper adenovirus. Preferably, AV E1a and E1b genes are integrated into the host cell genome (e.g. as in HEK293 cells) in both first and second host cells.

As mentioned above, the AAV *rep* genes and/or the first AAV cap gene may independently be introduced (e.g. transfected) into the host cells in one or more recombinant adenoviruses or be present in one or more recombinant adenoviruses which are already in the host cell.

In order to accommodate the AAV *rep* gene and/or the AAV *cap* gene, part or all of one or more adenoviral genes may be deleted (preferably genes which are not adenoviral helper genes). For example, the AAV *rep* gene and/or the AAV *cap* gene may be inserted into one of the adenoviral Early genes or inserted in a site from which Early genes have been deleted from an adenovirus. In the latter example, the deleted Early genes may be trans-complimented by a cell line containing the deleted genes, e.g. HEK293 cells which contain the adenoviral E1A and E1B regions.

The AAV rep gene and/or the AAV cap gene may be inserted into a region of an adenoviral genome containing an E1 deletion. In other instances, genes that are non-essential to the adenovirus can also be deleted and these sites can be used to insert a nucleic acid molecule of the invention. For example, the AAV rep gene and/or the AAV cap gene may be inserted in the E3 region of an adenovirus because most E3 genes can be deleted in an adenoviral vector.

The AAV rep gene and/or the AAV cap gene may be inserted into an adenoviral gene in sense or antisense orientation (with respect to the direction of transcription of the adenoviral gene). It is a preferred embodiment of the invention that the AAV rep gene and/or the AAV cap gene will be in the same direction of transcription as the E4, E2A and E2B expression cassettes when it is inserted into the E1 region. This is to prevent the E1A promoter (that is often retained in E1-deleted AV's) from acting as a promoter to drive the rep gene expression. The E1A promoter cannot be removed because it contains the AV packaging signal.

It is a preferred embodiment of the invention that the Rep-coding sequence will not contain an upstream promoter.

In some preferred embodiments, the AAV rep gene and/or the second AAV cap gene is inserted into an adenoviral E1 gene, preferably wherein part of the E1 gene has been deleted. Preferably, the E1 gene is E1A and/or E1B.

The *rep* genes, the *cap* genes and the viral helper genes may each independently be operably-associated with a promoter, e.g. a constitutive promoter, an inducible promoter, a repressible promoter, a minimal promoter, or with no promoter.

As used herein, the term "operably-associated" in the context of a promoter and a gene means that the promoter and the gene in question are located within a distance from each other which is sufficiently close for the promoter to promote transcription of the gene. In some embodiments, the promoter and the gene are juxtaposed or are contiguous.

Preferably, the promoters which are operably-associated with the first and/or second AAV cap genes are constitutive or inducible promoters, more preferably constitutive promoters, such as a minimal CMV promoter.

In some embodiments, the first and/or second AAV cap gene is operably-associated with no promoter or with a minimal promoter.

Preferably, the promoter which is operably-associated with the first AAV cap gene (in the recombinant AAV genome) is an inducible promoter or a repressible promoter. Such a promoter may contain a 4C5G binding site that is bound by a modified TetR protein that does not bind a normal Tet operator site such as that described by Krueger *et al.* (Krueger et al., Gene, vol. 404, Issues 1-2, 1 December 2007, pages 93-100).

The 4C5G binding site (tccccgtcagtgacggaga, SEQ ID NO: 31) can be inserted into a promoter that contains a TATA box by placing the binding site in between the TATA box and the transcription start site (TSS). An example of this would be a CMV promoter containing two4C5G binding sites such as in the following sequence (wherein the TATA box and two 4C5G binding sites are shown in bold):

tagtaatcaattacggggtcattagttcatagcccatatatggagttccgcgttacataacttacggtaaatggcccgcctggc
tgaccgcccaacgacccccgcccattgacgtcaataatgacgtatgttcccatagtaacgccaatagggactttccattga
cgtcaatgggtggagtatttacggtaaactgcccacttggcagtacatcaagtgtatcatatgccaagtacgccccctattga
cgtcaatgacggtaaatggcccgcctggcattatgcccagtacatgaccttatgggactttcctacttggcagtacatctacg
tattagtcatcgctattaccatgctgatgcggttttggcagtacatcaatgggcgtggatagcggtttgactcacggggatttcc
aagtctccaccccattgacgtcaatgggagtttgttttggcaccaaaatcaacgggactttccaaaatgtcgtaacaactcc
gccccattgacgcaaatgggcggtaggcgtgtacggtgggaggtctatataagcagagctgtccccgtcagtgacgg
agatgtccccgtcagtgacggagatcgtcgagcagctcgtttagtgaacc (SEQ ID NO: 32).

The 4C5G site can be bound by a repressor called TetR-V36A-E37A-P39K which has the following coding sequence:

atgtcgcgcctggacaaaagcaaagtgattaactcagcgctggaactgttgaatgaggtgggaattgaaggactcactac
tcgcaagctggcacagaagctgggcgccgcgcagaagacgctgtactggcatgtgaagaataaacgggcgctcctag
acgcgcttgccatcgaaatgctggaccgccatcacacccacttttgccccctggagggcgaatcctggcaagattttctgc
ggaacaatgcaaagtcgttccggtgcgctctgctgtcccaccgcgatggcgcaaaagtgcacctgggcactcggcccac
cgagaaacaatacgaaaccctggaaaaccaactggctttcctttgccaacagggattttcactggagaatgccctgtacg
cactatccgcggtcggccactttaccctgggatgcgtcctcgaagatcaggagcaccaagtcgccaaggaggaaagag
aaactcctaccactgactcaatgcctccgctcctgagacaagccatcgagctgttcgaccaccagggtgctgaacctgcat
ttctgttcgggcttgaactgattatctgcggcctggagaaacagttgaagtgcgagtcgggatcctag (SEQ ID NO:
33).

Other repressors and appropriately-paired repressor binding sites can be used instead of the example embodied here and such alternatives are well known to those in the art.

Alternatively, the promoter operably-associated with the first AAV cap gene (which drives expression of the first Cap polypeptides) may not be active in the first host cells because of the absence of an activator protein that is required to bind the promoter to initiate transcription for the first cap gene. An appropriate activator would be the Cumate activator protein that binds a cumate binding site (e.g. AGAAACAAACCAACCTGTCTGTATTA, SEQ ID NO: 34) that is a modified form of the Cumate repressor that contains an activator domain.

The following sequence is an example of a minimal CMV promoter containing Cumate activator binding sites. The promoter will only express in the presence of a Cumate activator protein.

GATCTGTCGACCAACTAGTACCccgggAACTCGTCACGACGAAAGAAACAAACCAA
CCTGTCTGTATTATCAAAGAAACAAACCAACCTGTCTGTATTATCAAAGAAACAAA
CCAACCTGTCTGTATTATCAAAGAAACAAACCAACCTGTCTGTATTATCAAAGAAA
CAAACCAACCTGTCTGTATTATCAAAGAAACAAACCAACCTGTCTGTATTATCcgA

AGGTAGGCGTGTACGGTGGGAGGTCTATATAAGCAGAGCTGGTTTAGTGAACCGT
CAGATCA (SEQ ID NO: 35).

The Cumate activator (CmyR-VP16) protein coding sequence can be found here:

Atgagtccaaagagaagaacacaggcagagcgcgcaatggagacccagggcaagttgattgcagcggccctgggg
gttttacgggaaaaaggttacgcgggattccggatcgcagatgtgcccggtgctgcaggtgtctcgagaggagcgcaga
gccatcatttcccgacaaagcttgagcttctgcttgccacttttgaatggctttacgaacagatcaccgaacgcagtcgggct
cgattagcgaaattgaagccagaggatgacgtcatccagcaaatgctggacgacgccgccgaatttttcctcgacgatga
cttctctatcagccttgatttgattgtggctgccgaccgggatccagcgttacgcgagggtattcagcgcacggtagagagg
aatcggtttgtcgtcgaggatatgtggcttggtgttctggtgagccgtggtctttcgcgtgatgatgcagaagatatcctttggtt
gatattcaattcggtgcgtgggcttgctgttcgtagcctatggcagaaggacaaagaacgctttgagcgtgtcaggaactcg
acactcgaaattgcgcgagagcggtacgcgaaattcaagcgctaCccgaccgacgcccttgacgattttgacttagacat
gctcccagccgatgcccttgacgactttgaccttgatatgctgcctgctgacgctcttgacgattttgaccttgacatgctcccc
gggtaaTAg (SEQ ID NO: 36).

Other activator proteins and appropriately-paired activator binding sites can be used to achieve the same objective as the example embodied here and such combinations are well known to those in the art.

As used herein, the terms "activator" and "inducer" are used interchangeably. Preferably, when the promoter which is operably-associated with the AAV cap gene is encoded within a recombinant AV, the promoter used will be selected based on the toxicity of the cap gene to the adenovirus and the expression levels required. The inventors have found that some cap genes can be expressed under the CMV promoter and have little to no impact on AV replication, e.g. AAV9. Conversely, some cap genes can only be inserted into AVs if driven by a minimal CMV promoter that has comparatively low expression, e.g. AAV6. Therefore, the promoter driving the cap gene will preferably be based on experimentally testing a low- and high-expressing promoter, where the high-expressing promoter is the CMV promoter and the low expression is the minimal promoter region from the same promoter.

Preferably, the *rep* gene promoter is the SV40 promoter, or a promoter which is derived therefrom, or a promoter of equal or decreased strength compared to the SV40 promoter in human cells and human cell lines (e.g. HEK-293 cells).

In some embodiments, the Rep polypeptide is expressed at a low, baseline or minimal level. In some embodiments, the AAV *rep* gene is not operably-associated with any functional promoter. As used herein, the term "low, baseline or minimal level" refers to a level of expression of the Rep78 polypeptide which is less than 50%, 40%, 30%, 20% or 10% of the level of expression of a wild-type Rep 78 polypeptide which is operably-associated with a wild-type p5 promoter (in a wild-type AAV rep gene).

In this way, sufficient Rep polypeptide is provided in order to enable the production of at least some AAV, but the level of Rep polypeptide expression is insufficient to completely inhibit adenovirus replication.

Preferably, the promoters which are operably-associated with the helper genes are constitutive or inducible promoters, more preferably constitutive promoters. Examples of constitutive promoters include the CMV, SV40, PGK (human or mouse), HSV TK, SFFV, Ubiquitin, Elongation Factor Alpha, CHEF-1, FerH, Grp78, RSV, Adenovirus E1A, CAG or CMV-Beta-Globin promoter, or a promoter derived therefrom.

In some embodiments, the promoter is inducible or repressible by the inclusion of an inducible or repressible regulatory (promoter) element. For example, the promoter may be one which is inducible with doxycycline, tetracycline, IPTG or lactose.

The *rep* genes and the *cap* genes may each also independently be operably-associated with a terminator, e.g. an SV40 polyadenylation signal.

Step (b) comprises culturing the first host cells under conditions such that first recombinant AAV particles are produced which are encapsidated by second Cap polypeptides. Conditions for the culturing of host cells for the production of AAVs are well known in the art. During the culturing step, AAV Rep polypeptides and second AVV Cap polypeptides will be produced, and also viral helper polypeptides. No first AAV Cap polypeptides are produced in the first host cells. The recombinant AAV genome will be replicated and encapsidated by the second Cap (capsid) polypeptides, thus producing first recombinant AAV particles.

Step (c) comprises infecting a population of second host cells with first recombinant AAV particles. The first recombinant AAV particles will be encapsidated by (second) AAV capsid polypeptides which confer a tropism towards second host cells. The second host cells are infected in this step with first recombinant AAV particles. The first recombinant AAV particles which are used in Step (c) are preferably purified or substantially first recombinant AAV particles.

Preferably, the second host cells are infected with the first recombinant AAV particles at a low multiplicity of infection (MOI), preferably at an MOI of less than one viral particle per cell. This is in order to probabilistically increase the chances that each host cell is only infected with one virus particle. This aims to ensure that all of the virus particles which are produced within any one cell that has been infected by one first AAV particle are encapsidated by Cap polypeptides from a single recombinant AAV genome that encodes one first AAV capsid gene.

The second host cells are cells towards which the first recombinant AAV particles have a tropism. The aim of Steps (c)-(d) is to produce a library of second recombinant AAV particles. Preferably, the second host cells are from an AAV production cell line or an AAV manufacturing cell line, i.e. a cell line which comprises all of the polypeptides which are necessary for AAV capsid production and AAV particle maturation. The second host cells may comprise one or more different types of cells (e.g. a mixture of HEK293 and PerC6 cells) or all be of the same type. The second host cells may be any of the types of first host cells, as defined herein. Preferably the second host cells are HEK293 cells or derivatives thereof.

Step (d) comprises culturing the second population of host cells in a culture medium under conditions such that:
(i) first AAV Cap polypeptides are produced (i.e. by expression of the recombinant AAV genome), and
(ii) AAV *rep* gene and viral helper genes are expressed in the second host cells,

wherein, if the first AAV cap gene is operably-associated with an inducible promoter, the inducer is present in the second population of host cells,
wherein the AAV *rep* gene is present in the genome of a recombinant adenovirus which is present in the second population of host cells,
wherein an AAV particle library of second recombinant AAV particles is produced,
wherein each second recombinant AAV particle in the library comprises a recombinant AAV genome which comprises ITRs flanking a first AAV *cap* gene encoding a first Cap polypeptide, wherein the particles in the library differ in the nucleotide sequences of their first AAV cap genes, and wherein each particle in the library is encapsidated by Cap polypeptides which are encoded by the (first) *cap* gene in its AAV genome.

In Step (d), the first AAV *cap* gene (in the recombinant AAV genome) is expressed, i.e. its expression is not prevented or repressed. The second AAV *cap* gene is preferably not expressed in the second host cells. An AAV *rep* gene and viral helper genes are expressed in the second host cells in order to facilitate the replication of the recombinant AAV genome.

The recombinant AAV genome will be replicated and encapsidated by the first Cap (capsid) polypeptides, thus producing second recombinant AAV particles.

The AAV *rep* gene is as defined above. The nucleotide sequences of the AAV *rep* gene used in Steps (a) and (c) may be the same or different.

The viral helper genes are as defined above. The viral helper genes used in Steps (a) and (c) may be the same or different.

Preferably, all or the majority of second host cells each independently produce recombinant AAV particles all of which comprise the same recombinant AAV genome within that cell (i.e. in any one cell, all of the recombinant AAV particles which are produced by that cell are the same).

Conditions for the culturing of host cells for the production of AAVs are well known in the art. The host cells will, in general, be cultured in a culture medium, preferably a liquid culture medium.

In this way, second recombinant AAV particles are produced which are encapsidated by first AAV capsid polypeptides, thus producing recombinant AAV particles with a range of tropisms towards third host cells.

Step (e) is optional. It comprises purifying and/or isolating a library of second recombinant AAV particles from the second host cells or from the culture medium. Methods of purifying and/or isolating recombinant AAV particles from host cells and culture media are well known in the art.

In certain embodiments, the library of recombinant AAV particles may comprise at least 10⁴, 10⁵, 10⁸, 10⁷, 10⁸, 10⁹ or more different recombinant AAV particles. As will be understand by those of skill in the art, there may be some particles in the library which have the same AAV cap gene sequence.

In the process of the invention, a library of recombinant AAV particles is produced, the AAV particles being encapsidated by a variety of different capsids, thus conferring a variety of different tropisms towards (third) host cells.

The library may be used in screening for recombinant AAV particles which have a specificity or an enhanced specificity for defined (third) host cells.

The first AAV capsid polypeptides confer a tropism on the second recombinant AAV particles towards third host cells (target cells). Preferably, the third host cells are ones which do not have the same AAV receptors as high-efficiency producer/manufacturing cell lines (e.g. HEK293, PerC6, 911). Preferably, the third host cells are not of the same cell type as either the first or second host cells.

Preferred third host cells (target cells) include but are not limited to neurons (preferably retinal neurons), hepatocytes, muscle cells, stem cells (e.g. haematopoietic stem cells, mesenchymal stem cells, embryonic stem cells, adipose stem cells, induced pluripotent stem cells, and their derivatives), immune cells (including B and T lymphocytes, natural killer cells, monocytes and macrophages and granulocytes), endothelial cells, cardiovascular cells, epithelial cells, mesenchymal cells, pancreatic b cells or pancreatic a cells, cardiomyocytes, spleen cells, fat cells, glial cells, fibroblasts, Kupffer cells and cancer cells (e.g. leukaemia, lymphoma, myeloma, carcinoma, sarcoma, melanoma cells).

Preferably, the process steps are carried out in the order specified.

WO2019/020992 discloses that transcription of the Late adenoviral genes can be regulated (e.g. inhibited) by the insertion of a repressor element into the Major Late Promoter. By "switching off" expression of the adenoviral Late genes, the cell's protein-manufacturing capabilities can be diverted toward the production of a desired recombinant protein or recombinant AAV particles.

In some embodiments, therefore, the recombinant adenovirus (i.e. adenoviral vector) comprises a repressible Major Late Promoter (MLP) and a plurality of adenoviral late genes, wherein the MLP comprises one or more repressor elements which are capable of regulating or controlling transcription of the adenoviral late genes, and wherein one or more of the repressor elements are inserted downstream of the MLP TATA box.

In other embodiments, the recombinant adenovirus (i.e. adenoviral vector) comprises
(a) a plurality of adenoviral early genes, and
(b) a plurality of adenoviral late genes under the control of a Major Late Promoter (MLP), and
(c) a transgene (e.g. comprising AAV *rep* and cap genes),
wherein the MLP comprises one or more repressor elements which are capable of regulating or controlling transcription of the adenoviral late genes, and wherein one or more of the repressor elements are inserted downstream of the MLP TATA box.

Preferred features for producing viral (preferably AAV) particles include the following:
- wherein the one or more repressor elements are inserted between the MLP TATA box and the +1 position of transcription.
- wherein the repressor element is one which is capable of being bound by a repressor protein.
- wherein a gene encoding a repressor protein which is capable of binding to the repressor element is encoded within the adenoviral genome.
- wherein the repressor protein is transcribed under the control of the MLP.
- wherein the repressor protein is the tetracycline repressor, the lactose repressor or the ecdysone repressor, preferably the tetracycline repressor (TetR).
- wherein the repressor element is a tetracycline repressor binding site comprising or consisting of the sequence set forth in SEQ ID NO: 28.
- wherein the nucleotide sequence of the MLP comprises or consists of the sequence set forth in SEQ ID NO: 29 or 30.
- wherein the presence of the repressor element does not affect production of the adenoviral E2B protein.
- wherein the adenoviral vector encodes the adenovirus L4 100K protein and wherein the L4 100K protein is not under control of the MLP.
- wherein a transgene is inserted within one of the adenoviral early regions, preferably within the adenoviral E1 region instead of in a Transfer Plasmid.

Preferably, one or more of the repressor elements are inserted downstream of the MLP TATA box.

Preferably, the transgene comprises AAV *rep* and cap genes.

There are many established algorithms available to align two amino acid or nucleic acid sequences. Typically, one sequence acts as a reference sequence, to which test sequences may be compared. The sequence comparison algorithm calculates the percentage sequence identity for the test sequence(s) relative to the reference sequence, based on the designated program parameters. Alignment of amino acid or nucleic acid sequences for comparison may be conducted, for example, by computer-implemented algorithms (e.g. GAP, BESTFIT, FASTA or TFASTA), or BLAST and BLAST 2.0 algorithms.

Percentage amino acid sequence identities and nucleotide sequence identities may be obtained using the BLAST methods of alignment (Altschul et al. (1997), "Gapped BLAST and PSI-BLAST: a new generation of protein database search programs", Nucleic Acids Res. 25:3389-3402; and http://www.ncbi.nlm.nih.gov/BLAST). Preferably the standard or default alignment parameters are used.

Standard protein-protein BLAST (blastp) may be used for finding similar sequences in protein databases. Like other BLAST programs, blastp is designed to find local regions of similarity. When sequence similarity spans the whole sequence, blastp will also report a global alignment, which is the preferred result for protein identification purposes. Preferably the standard or default alignment parameters are used. In some instances, the "low complexity filter" may be taken off.

BLAST protein searches may also be performed with the BLASTX program, score=50, wordlength=3. To obtain gapped alignments for comparison purposes, Gapped BLAST (in BLAST 2.0) can be utilized as described in Altschul et al. (1997) Nucleic Acids Res. 25: 3389. Alternatively, PSI-BLAST (in BLAST 2.0) can be used to perform an iterated search that detects distant relationships between molecules. (See Altschul et al. (1997) supra). When utilizing BLAST, Gapped BLAST, PSI-BLAST, the default parameters of the respective programs may be used.

With regard to nucleotide sequence comparisons, MEGABLAST, discontiguous-megablast, and blastn may be used to accomplish this goal. Preferably the standard or default alignment parameters are used. MEGABLAST is specifically designed to efficiently find long alignments between very similar sequences. Discontiguous MEGABLAST may be used to find nucleotide sequences which are similar, but not identical, to the nucleic acids of the invention.

The BLAST nucleotide algorithm finds similar sequences by breaking the query into short subsequences called words. The program identifies the exact matches to the query words first (word hits). The BLAST program then extends these word hits in multiple steps to generate the final gapped alignments. In some embodiments, the BLAST nucleotide searches can be performed with the BLASTN program, score=100, wordlength=12.

One of the important parameters governing the sensitivity of BLAST searches is the word size. The most important reason that blastn is more sensitive than MEGABLAST is that it uses a shorter default word size (11). Because of this, blastn is better than MEGABLAST at finding alignments to related nucleotide sequences from other organisms. The word size is adjustable in blastn and can be reduced from the default value to a minimum of 7 to increase search sensitivity.

A more sensitive search can be achieved by using the newly-introduced discontiguous megablast page (www.ncbi.nim.nih.gov/Web/Newsitr/FallWinter02/blastlab.html). This page uses an algorithm which is similar to that reported by Ma et al. (Bioinformatics. 2002 Mar; 18(3): 440-5). Rather than requiring exact word matches as seeds for alignment extension, discontiguous megablast uses a non-contiguous word within a longer window of template. In coding mode, the third base wobbling is taken into consideration by focusing on finding matches at the first and second codon positions while ignoring the mismatches in the third position. Searching in discontiguous MEGABLAST using the same word size is more sensitive and efficient than standard blastn using the same word size. Parameters unique for discontiguous megablast are: word size: 11 or 12; template: 16, 18, or 21; template type: coding (0), non-coding (1), or both (2).

In some embodiments, the BLASTP 2.5.0+ algorithm may be used (such as that available from the NCBI) using the default parameters.

In other embodiments, a BLAST Global Alignment program may be used (such as that available from the NCBI) using a Needleman-Wunsch alignment of two protein sequences with the gap costs: Existence 11 and Extension 1.

The nucleic acid molecules, plasmids and vectors of the invention may be made by any suitable technique. Recombinant methods for the production of the nucleic acid molecules and production cell lines of the invention are well known in the art (e.g. "Molecular Cloning: A Laboratory Manual" (Fourth Edition), Green, MR and Sambrook, J., (updated 2014)).

The present invention is further illustrated by the following Examples, in which parts and percentages are by weight and degrees are Celsius, unless otherwise stated. It should be understood that these Examples, while indicating preferred embodiments of the invention, are given by way of illustration only.

### General methods

### Recovery of adenoviral vectors containing AAV components

All recombinant adenoviral vectors have been initially recovered in HEK293 cells from plasmid DNA encoding each viral genome. Plasmids (20 µg) were linearised with Swal restriction enzymes to release virus ITRs from the bacterial plasmid backbone and purified using genomic Purelink DNA extraction kit (Invitrogen, CA, USA). HEK293 cells were seeded in T25 tissue culture flasks, at a density of 2x10⁶ cells per flask, for 24-hours before transfection. Each flask was transfected with 2.5 µg of linearised DNA using Lipofectamine 2000 (Invitrogen) according to the manufacturer's protocol. Transfection media were exchanged with fresh DMEM containing 2% FBS (supplemented with doxycycline 0.5 µg/mL or DMSO) after 4 hours. Recombinant viruses were harvested from growth media ~12 days post-transfection upon observation of full CPE. Single clone was isolated by two-rounds of serial dilution and further propagated in HEK293 cells (and cultured with doxycycline 0.5 µg/mL or DMSO). Viruses were harvested by three rounds of freeze-thaw at day 3 post-infection. Cellular debris were pelleted by centrifugation and supernatant passed through a 0.2 µm filter. For large scale virus amplification and purification, HEK293 cells were seeded in Corning^{®} HYPERFlask^{®} (Sigma-Aldrich, MO, USA) for 48-hours so that they are ~95% confluent before infection. For adenoviral vectors containing rep and cap genes viral stock was used to infect each HYPERFlask at an MOI of 3 for 3 days. For adenoviral vectors containing a modified major late promoter that is regulated by the tetR protein, cell cultures were supplemented with doxycycline 0.5 µg/mL. Cells are harvested upon display of full CPE and virus released by three rounds of freeze-thaw. Virus were purified by two rounds of CsCl gradient banding, with Benzonase 250 U/mL (Sigma-Aldrich, MO, USA) added after the first round to degrade free DNA.

### AAV transduction assay

Transduction-competent AAV can be measured using a modified TCID50 assay. HEK293 cells can be seeded in 96-well tissue culture plates at a density of 1x10⁴ cells per well for 24-hours. Eight 10-fold serial dilutions of AAV crude lysate stock can then be made in DMEM containing 2% FBS at a total volume of 1.2mL. Ten replicates of each diluted sample (1x10⁻² to 1x10⁻¹⁰) can then be added at a volume of 100uL per well on each plate. 100uL of DMEM containing 2% FBS can be added to the final two columns as negative control. Plates can then be observed for the presence of EGFP expressing cells from each well 96hpi using a fluorescent microscope (EVOS FL imaging system, ThermoFisher Scientific, MA, USA). Transducing units per mL can be calculated using KÄRBER-SPEARMAN statistical method.

### Quantification of viral genomes and gene expression using qPCR

For quantification of total adenovirus genomes in HEK293 cells, total DNA was extracted from culture media and cellular lysates using Purelink genomic DNA miniprep kit (Invitrogen, CA, USA). Five microlitres of DNA eluent were used in qPCR reactions using TaqMan Fast Advanced Master Mix (Applied Biosystems, CA, USA) in a StepOnePlus Real-Time PCR System (Applied Biosystems, CA, USA).

For quantification of genome encapsulated AAV and adenovirus particles, 2 µL of viral samples, harvested from culture medium or cell lysates, were treated with 1U of TURBO DNase (ThermoFisher Scientific, MA, USA) in a 20 µL reaction for 2-hours at 37°C. TURBO DNase was heat-inactivated at 75°C for 10-minutes. Five microlitres of samples diluted at 1:200 using nuclease-free water were used in the PCR reaction to quantify encapsulated Ad5 using Ad5 hexon primers and probe, while EGFP primers and probe were used to quantify encapsulated AAV genomes. Titres of total AAV vectors produced using helper adenovirus were determined by subtraction of genome encapsulated adenoviruses. Standard curves for qPCR analyses were generated using a gBLOCK gene fragment suspended in nuclease-free water (Integrated DNA Technologies, IA, USA) and CT values of PCR reaction used to calculate DNA copy number by extrapolation to the standard curves (a qPCR standard of 3x10⁸-3x10¹ copies/well).

### AAV capsid quantification by ELISA

Detection of assembled AAV2 capsids can be carried out using AAV2 titration ELISA kit (Progen, Heidelberg, Germany) according to the manufacturer's instruction.

### Example 1: Production of DNA library

The DNA molecules containing the first capsid library variants can be amplified in a pool by PCR using primers flanking the variable capsid region, or the entire capsid gene. If the amplified capsid library pool is to be inserted by ligation, the ends of the amplified DNA molecules can be digested using restriction enzymes using standard molecular biology approaches. If the amplified capsid library is to be inserted by Gibson assembly, the amplified fragment should be first gel purified. The method of assembly will have been decided during the design of the library.

For libraries to be assembled by ligation, prepare a ligation mixture on ice by combining 2 µL of the gel purified library, 50ng of digested plasmid vector, 5 µL of Electroligase buffer and 1 µL of Electroligase enzyme. Make the total reaction volume up to 11 µL with nuclease free water. Mix the reaction by pipetting it up and down 4 times. Place the reactions in a thermocycler and run a program that will incubate the reactions at 25°C for 60 minutes followed by 65°C for 15 minutes. Once the program has completed, store the reactions in a freezer until required. The size of the ligation should be increased according to the library size. For a library of 20,000 variants approximately 72 µL of ligation reaction will be required and the ligation reaction components can be scaled accordingly and proportionally.

For libraries to be assembled by Gibson assembly, set up the assembly reactions in PCR tubes on ice and mix them well by pipetting up and down 4 times. Prepare the reaction by combining 0.1 pMol of Capsid library DNA fragment bearing homology to the digested plasmid vector with 0.02 pMol of digested plasmid (recipient vector) with 10 µL of 2x Gibson assembly master mix then make up the reaction volume to 20 µL. Place the reactions in a thermocycler and incubate them at 50°C for 60 minutes. Once the program has completed, add 40 µL of nuclease free water to each tube and mix by pipetting up and down 4 times. Label the tubes with sample name, initials and date. Store the reactions in a -20 degree freezer until required.

For a 20,000-variant capsid library, perform 12 x transformations using 6 µL of ligation reaction and 200 µL of electrocompetent E. *coli* cell suspension for each (plus a transformation using 1.5 µL of the 'no insert' control ligation plus 50 µL cells). DNA transformations can be achieved via electroporation using the protocol recommended by the electroporation manufacturer or the provider of the electrocompetent cells. All transformations can then be pooled into one tube at the end and the samples spread on 48 LB agar 160 mm plates.

Count the number of colonies on three plates to calculate the average colonies per plate. There should be no colonies on the 'no insert' plate.

Multiply this by the number by 37,600 to obtain the number of colony forming units (CFUs) in the library. Note that this number is calculated here for a 20,000 variant library; the multiplier will vary according to the library size and would need to be calculated by the user on each occasion. Select 24 colonies for culturing and purify plasmid DNA and sequence the 24 plasmid samples using an appropriate primer by Sanger sequencing. If at least 75% of the reads contain inserts and are known to exist in the capsid library, then the library can be considered to have passed quality control. For example, if the mean number of colonies is 140 in a 20,000-variant library, the number of CFUs in the whole library would be approximately 140 x 37,600 = 5,264,000. This gives an indication of the quality of the library and should be at least 30 x the number of variants in the library to provide good coverage of all variants.

If the library has passed the Sanger sequencing QC step, prepare a sample of the library DNA for next generation sequencing. If the data conforms to the following criteria, the library can be considered to have passed the NGS QC:
- Uniformity (at least 90% within 10-fold range)
- Dropouts (lower than 5%)
- Runaways (the most abundant 1% should be lower than 10% of the total number of reads)

Capsid libraries that have passed QC can be frozen at -20 degrees until required for testing.

### Example 2: Production of AAV in first host cells via either plasmid or adenoviral approaches to produce first AAV particles encapsulated second AAV capsids and encoding first AAV capsids

This example assumes the method of regulation of the first AAV capsid genes is that the promoter driving expression of the first capsids requires an activator protein that is not in the first host cells used in this example. For the production of first AAV particles encapsulated in AAV2 capsids (second AAV capsids) that encode the first AAV capsids, production of the AAV can be achieved in a range of formats that are suitable to the library size being produced. For a small library of approximately 10⁴ variants, 6-well tissue culture treated plates can be used. Briefly, HEK293 cells can be seeded at a density of 7.5x10⁴ cells per well for 24 hours prior to transfection. For helper-free production, each well can be transfected with 2.5 µg of plasmid DNA, containing pHelper, pRepCap2 and a library of DNA molecules that contain two AAV ITRs flanking the Cap variants that constitute first AAV capsids. These plasmids can be diluted with Opti-MEM (Gibco, MA, USA) at a DNA mass ratio of 1:1:1, and complexed using linear PEI 25kDA (Polysciences) at a 1:3 DNA to PEI mass ratio.

For the production of first AAV particles encapsulated in AAV2 capsids (second AAV capsids) that encode the first AAV capsids, production can also be achieved using adenoviral vectors containing AAV *rep* and cap genes. For a small capsid library of approximately 10⁴ variants, 6-well tissue culture treated plates can be used. Briefly, HEK293 cells can be seeded at a density of 7.5x10⁴ cells per well for 24 hours prior to transfection. Each well can then be infected with AV containing the Rep and Cap2 genes whilst also being transfected with 0.75µg of a library of DNA molecules that contain two AAV ITRs flanking the Cap variants that constitute first AAV capsids. Infection/transfection media can be exchanged with fresh DMEM containing 2% FBS (supplemented with doxycycline 0.5 µg/mL or DMSO) 4 hours post treatment.

AAV vectors can be harvested via three rounds of freeze-thaw of cells suspended in growth media. Cells can be pelleted by centrifugation at 3000g for 20 minutes and supernatant passed through a 0.2 µm filter prior to analysis or use to infect second host cells.

### Example 3: Infection of second host cells using first AAV particles encapsulated by second AAV capsids to produce second AAV particles that are encapsulated by first AAV capsids

First AAV particles encoding the first AAV capsids can be used to infect HEK293 cells alongside co-infection with a Tetracycline-enabled repressible adenovirus encoding the AAV Rep gene (TERA-Rep). In this example, HEK293 cells can be seeded in 6-well tissue culture plate format at 0.5x10⁶ per well 24 hours prior to infection. HEK293 cells can then be co-infected by simple pipetting with both TERA-Rep at 5 infectious particles per cell and the first AAV particles at 0.1 genome copies per cell to maximise the number of cells receiving only one AAV particle.

Second AAV vectors encapsulated in first AAV capsids can be harvested 96 hours post-infection via three rounds of freeze-thaw of cells suspended in growth media. Cells can be pelleted by centrifugation at 3000g for 20 minutes and supernatant passed through a 0.2 µm filter prior to analysis or use to infect third host cells. Encapsulated AAV particles can be quantified by QPCR as described above.

As the first AAV capsids will have novel binding properties, it is likely that material isolated from the crude cell lysate would be used to infect third host cells for screening purposes because using capsid affinity based purification methods would potentially skew the library towards those having a greater affinity for the resin used. Alternatively, for larger libraries, AAV particles can be purified via lodixanol or CsCl density centrifugation. Such methods of purification are well known to those in the art.

### Example 4: Infection of third host (target) cells using the second AAV particles encapsulated by first AAV capsids to isolate AAV particles with improved tropism towards third host cells

Target cells can be transduced with AAV from a second host cell. This is achieved through a range of approaches that are suitable for the cell type being investigated.

Delivery to third host cells can be achieved via direct *in vivo* delivery by needle injection to a suitable animal model e.g. via intravenous tail vein injection using a 28G or 30G needle and injecting no more than 250µl with the AAV particles resuspended in phosphate buffered saline. In this example, after 72 hours the mice may be dispatched and the appropriate target organ can then be assessed. This may involve the analysis of reporter gene expression in the target organ where the second AAV particles also encoded such a reporter gene. In order to do this efficiently, it may be required that the tissue is dissociated using an appropriate method such as using a gentleMACS dissociator (Miltenyi Biotec). Individual cells can be analysed using flow cytometry and where a reporter gene is encoded in the second AAV particles fluorescent activated cell sorting can be used to identify and isolate cells containing specific first AAV capsid sequences of interest. These can then be sequenced using many sequencing approaches well known to those in the art (e.g. PCR amplification of the capsid gene followed by Sanger sequencing, or nanopore sequencing, or Illumina next generation sequencing).

Delivery to third host cells can also be achieved using primary cells ex *vivo,* e.g. T cells derived from peripheral blood mononuclear cells (PBMCs) from a human. Cells where specific first AAV capsids endow improved tropism can then be identified and isolated as described above and capsid sequences can be analysed using the same technologies. Delivery to third party cells can also involve the infection of second AAV particles into a cell line *in vitro.* Where the second AAV particles contain a reporter gene the cells that contain capsid sequences that endow improved transduction upon second AAV particles can be identified and sequenced in the same way as described above.

### SEQUENCE LISTING FREE TEXT

<210> 1 <223> AAV1 - Capsid Nucleotide Sequence (AF063497.1)
<210> 2 <223> AAV1 - Capsid Protein Sequence
<210> 2 <223> AAV1 - Capsid Protein Sequence
<210> 9 <223> AAV5 - Capsid Nucleotide Sequence (AF085716.1)
<210> 10 <223> AAV5 - Capsid Protein Sequence
<210> 11 <223> AAV6 - Capsid Nucleotide Sequence (AF028704.1)
<210> 12 <223> AAV6 - Capsid Protein Sequence
<210> 13 <223> AAV7 - Capsid Nucleotide Sequence (AF513851.1)
<210> 14 <223> AAV7 - Capsid Protein Sequence
<210> 15 <223> AAV8 - Capsid Nucleotide Sequence (AF513852.1)
<210> 16 <223> AAV8 - Capsid Protein Sequence
<210> 17 <223> AAV9 - Capsid Nucleotide Sequence (AY530556.1)
<210> 18 <223> AAV9 - Capsid Protein Sequence
<210> 28 <223> TetR binding site
<210> 29 <223> Modified MLP
<210> 30 <223> Modified MLP
<210> 31 <223> 4C5G binding site
<210> 32 <223> CMV promoter containing two 4C5G binding sites
<210> 33 <223> TetR-V36A-E37A-P39K coding sequence
<210> 35 <223> Minimal CMV promoter containing Cumate activator binding sites
<210> 36 <223> Cumate activator (CmyR-VP16) protein coding sequence

## Claims

1. A process for producing a library of recombinant AAV particles, the process comprising the steps:
(a) introducing, into a population of first host cells, a DNA library comprising a plurality of DNA molecules, wherein each DNA molecule in the library comprises a recombinant AAV genome which comprises ITRs flanking a first AAV cap gene encoding first Cap polypeptides, and wherein DNA molecules in the library differ in the nucleotide sequences of their first AAV *cap* genes;
(b) culturing the population of first host cells under conditions such that:
(i) a second AAV cap gene encoding second Cap polypeptides is expressed in the first host cells, wherein the second Cap polypeptides are ones which confer a tropism on AAV particles encapsidated by such polypeptides towards second host cells,
(ii) an AAV rep gene and viral helper genes are expressed in the first host cells, and
(iii) first AAV Cap polypeptides are not produced in the first host cells, by virtue of the expression of a repressor in the first host cells which represses or prevents expression of the first AAV Cap polypeptides or wherein the first AAV cap gene is operably-associated with an inducible promoter and the inducer is not present in the first host cells,
wherein the AAV rep gene and second cap gene are both present in the genome of a recombinant adenovirus which is present in the first host cells,
wherein first recombinant AAV particles are produced which are encapsidated by the second Cap polypeptides;
(c) infecting a population of second host cells with first recombinant AAV particles;
(d) culturing the second population of host cells in a culture medium under conditions such that:
(i) first AAV Cap polypeptides are produced, and
(ii) an AAV *rep* gene and viral helper genes are expressed in the second host cells,
wherein, if the first AAV *cap* gene is operably-associated with an inducible promoter, the inducer is present in the second population of host cells,
wherein the AAV *rep* gene is present in the genome of a recombinant adenovirus which is present in the second population of host cells,
wherein an AAV particle library of second recombinant AAV particles is produced, wherein each second recombinant AAV particle in the library comprises an AAV genome which comprises ITRs flanking a first AAV *cap* gene encoding a first Cap polypeptide, wherein the particles in the library differ in the nucleotide sequences of their first AAV *cap* genes, and wherein each particle in the library is encapsidated by Cap polypeptides which are encoded by the (first) *cap* gene in its AAV genome;
and optionally
(e) purifying and/or isolating a library of second recombinant AAV particles from the second host cells or from the culture medium.

2. A process as claimed in claim 1, wherein the DNA library is in the form of a library of plasmids or vectors, transposons, linear DNA molecules or lentiviral particles or lentiviral vectors.

3. A process as claimed in claim 1 or claim 2, wherein the first and/or second host cells are from an AAV production cell line or an AAV manufacturing cell line.

4. A process as claimed in any one of the preceding claims, wherein the first and/or second host cells are selected from the group consisting of HEK293, HEK293T, HEK293A, PerC6, 911 and HeLaRC32 cells.

5. A process as claimed in any one of the preceding claims, wherein the recombinant AAV genome additionally comprises a reporter gene.

6. A process as claimed in any one of the preceding claims, wherein the repressor is an antisense RNA, preferably a shRNA, a siRNA or a miRNA.

7. A process as claimed in claim 6, wherein the repressor is an antisense RNA which binds to the 5' or 3' UTR of a first AAV Cap mRNA, preferably to the 3' UTR.

8. A process as claimed in any one of the preceding claims, wherein the first AAV cap gene is operably-associated with a repressible promoter, the repressor is present in the first host cells, and the repressor is not present in the second host cells.

9. A process as claimed in any one of the preceding claims, wherein:
(i) the first recombinant AAV particles are of serotype 1, 2, 3, or 6; and/or
(ii) the second recombinant AAV particles are of serotype 9, or a derivative thereof.

10. A process as claimed in any one of the preceding claims, wherein the recombinant adenovirus comprises a repressible Major Late Promoter (MLP) and a plurality of adenoviral late genes, wherein the MLP comprises one or more repressor elements which are capable of regulating or controlling transcription of the adenoviral late genes, and wherein one or more of the repressor elements are inserted downstream of the MLP TATA box.

11. A process as claimed in any one of the preceding claims, wherein the second host cells are infected with first recombinant AAV particles at a low multiplicity of infection (MOI), preferably at a MOI of less than one viral particle per cell.

12. A process as claimed in any one of the preceding claims, wherein second AAV Cap polypeptides are not produced in the second host cells.

13. A process as claimed in any one of the preceding claims, wherein one or more of the second recombinant particles has a tropism towards third host cells, preferably a third host cell selected from the group consisting of neurons (preferably retinal neurons), hepatocytes, muscle cells, stem cells (preferably haematopoietic stem cells, mesenchymal stem cells, embryonic stem cells, adipose stem cells, or induced pluripotent stem cells, and their derivatives), immune cells (preferably B or T lymphocytes, natural killer cells, monocytes, macrophages or granulocytes), endothelial cells, cardiovascular cells, epithelial cells, mesenchymal cells, pancreatic b cells or pancreatic a cells, cardiomyocytes, spleen cells, fat cells, glial cells, fibroblasts, Kupffer cells, and cancer cells (preferably leukaemia, lymphoma, myeloma, carcinoma, sarcoma, or melanoma cells).

## Patentansprüche

1. Prozess zum Erzeugen einer Bibliothek rekombinanter AAV-Partikel, wobei der Prozess folgende Schritte umfasst:
a) Einbringen einer DNA-Bibliothek, die eine Vielzahl von DNA-Molekülen umfasst, in eine Population erster Wirtszellen, wobei jedes DNA-Molekül in der Bibliothek ein rekombinantes AAV-Genom umfasst, das ITRs umfasst, die ein erstes AAV-*Cap*-Gen flankieren, das erste Cap-Polypeptide codiert, und wobei sich die DNA-Moleküle in der Bibliothek in den Nukleotidsequenzen ihrer ersten AAV-*Cap*-Gene unterscheiden;
b) Kultivieren der Population erster Wirtszellen unter solchen Bedingungen, dass:
i) ein zweites AAV-Cap-Gen, das zweite Cap-Polypeptide codiert, in den ersten Wirtszellen exprimiert wird, wobei die zweiten Cap-Polypeptide diejenigen sind, die den von solchen Polypeptiden eingekapselten AAV-Partikeln einen Tropismus zu zweiten Wirtszellen verleihen,
ii) ein AAV-*Rep*-Gen und virale Helfergene in den ersten Wirtszellen exprimiert werden, und
iii) erste AAV-Cap-Polypeptide in den ersten Wirtszellen nicht produziert werden, aufgrund der Expression eines Repressors in den ersten Wirtszellen, der die Expression der ersten AAV-Cap-Polypeptide unterdrückt oder verhindert, oder wobei das erste AAV-*Cap*-Gen operativ mit einem induzierbaren Promotor verbunden ist und der Induktor in den ersten Wirtszellen nicht vorhanden ist,
wobei sowohl das AAV-*Rep*-Gen als auch das zweite Cap-Gen im Genom eines rekombinanten Adenovirus vorhanden sind, das in den ersten Wirtszellen vorhanden ist,
wobei erste rekombinante AAV-Partikel produziert werden, die von den zweiten Cap-Polypeptiden eingekapselt sind;
c) Infizieren einer Population zweiter Wirtszellen mit ersten rekombinanten AAV-Partikeln;
d) Kultivieren der zweiten Population von Wirtszellen in einem Kulturmedium unter solchen Bedingungen, dass:
i) erste AAV-Cap-Polypeptide produziert werden und
ii) ein AAV-*Rep*-Gen und virale Helfergene in den zweiten Wirtszellen exprimiert werden,
wobei, wenn das erste AAV-*Cap*-Gen operativ mit einem induzierbaren Promotor verbunden ist, der Induktor in der zweiten Population von Wirtszellen vorhanden ist,
wobei das AAV-*Rep*-Gen im Genom eines rekombinanten Adenovirus vorhanden ist, das in der zweiten Population von Wirtszellen vorhanden ist,
wobei eine AAV-Partikelbibliothek aus zweiten rekombinanten AAV-Partikeln erzeugt wird, wobei jedes zweite rekombinante AAV-Partikel in der Bibliothek ein AAV-Genom umfasst, das ITRs umfasst, die ein erstes AAV-Cap-Gen flankieren, das ein erstes Cap-Polypeptid kodiert, wobei sich die Partikel in der Bibliothek in den Nukleotidsequenzen ihrer ersten AAV-*Cap*-Gene unterscheiden, und wobei jede Partikel in der Bibliothek von Cap-Polypeptiden eingekapselt ist, die durch das (erste) *Cap*-Gen in seinem AAV-Genom kodiert werden;
und optional
e) Reinigen und/oder Isolieren einer Bibliothek zweiter rekombinanter AAV-Partikel aus den zweiten Wirtszellen oder aus dem Kulturmedium.

2. Prozess nach Anspruch 1, wobei die DNA-Bibliothek in Form einer Bibliothek von Plasmiden oder Vektoren, Transposonen, linearen DNA-Molekülen oder lentiviralen Partikeln oder lentiviralen Vektoren ist.

3. Prozess nach Anspruch 1 oder Anspruch 2, wobei die ersten und/oder die zweiten Wirtszellen aus einer AAV-Produktionszelllinie oder einer AAV-Herstellungszelllinie stammen.

4. Prozess nach einem der vorstehenden Ansprüche, wobei die ersten und/oder die zweiten Wirtszellen aus der Gruppe ausgewählt sind bestehend aus HEK293-, HEK293T-, HEK293A-, PerC6-, 911- und HeLaRC32-Zellen.

5. Prozess nach einem der vorstehenden Ansprüche, wobei das rekombinante AAV-Genom zusätzlich ein Reportergen umfasst.

6. Prozess nach einem der vorstehenden Ansprüche, wobei der Repressor eine Antisense-RNA, vorzugsweise eine shRNA, eine siRNA oder eine miRNA ist.

7. Prozess nach Anspruch 6, wobei der Repressor eine Antisense-RNA ist, die sich an die 5'- oder 3'-UTR einer ersten AAV-Cap-mRNA, vorzugsweise an die 3'-UTR bindet.

8. Prozess nach einem der vorstehenden Ansprüche, wobei das erste AAV-Cop-Gen operativ mit einem reprimierbaren Promotor verknüpft ist, der Repressor in den ersten Wirtszellen vorhanden ist und der Repressor in den zweiten Wirtszellen nicht vorhanden ist.

9. Prozess nach einem der vorstehenden Ansprüche, wobei:
(i) die ersten rekombinanten AAV-Partikel vom Serotyp 1,2,3 oder 6 sind; und/oder
(ii) die zweiten rekombinanten AAV-Partikel vom Serotyp 9 oder ein Derivat davon sind.

10. Prozess nach einem der vorstehenden Ansprüche, wobei das rekombinante Adenovirus einen unterdrückbaren Major Late Promoter (MLP) und eine Vielzahl von späten Adenovirusgenen umfasst, wobei der MLP ein oder mehrere Repressorelemente umfasst, die in der Lage sind, die Transkription der späten Adenovirusgene zu regulieren oder zu kontrollieren, und wobei ein oder mehrere der Repressorelemente stromabwärts der MLP-TATA-Box eingefügt sind.

11. Prozess nach einem der vorstehenden Ansprüche, wobei die zweiten Wirtszellen mit ersten rekombinanten AAV-Partikeln bei einer niedrigen Infektionsmultiplizität (MOI) infiziert werden, vorzugsweise bei einer MOI von weniger als einem Viruspartikel pro Zelle.

12. Prozess nach einem der vorstehenden Ansprüche, wobei in den zweiten Wirtszellen keine zweiten AAV-Cap-Polypeptide produziert werden.

13. Prozess nach einem der vorstehenden Ansprüche, wobei ein oder mehrere der zweiten rekombinanten Partikel einen Tropismus gegenüber dritten Wirtszellen aufweisen, vorzugsweise einer dritten Wirtszelle, die ausgewählt ist aus der Gruppe bestehend aus Neuronen (vorzugsweise retinalen Neuronen), Hepatozyten, Muskelzellen, Stammzellen (vorzugsweise hämatopoetischen Stammzellen, mesenchymalen Stammzellen, embryonalen Stammzellen, Fettgewebestammzellen oder induzierten pluripotenten Stammzellen und deren Derivaten), Immunzellen (vorzugsweise B- oder T-Lymphozyten, natürlichen Killerzellen, Monozyten, Makrophagen oder Granulozyten), Endothelzellen, kardiovaskulären Zellen, Epithelzellen, Mesenchymzellen, Pankreas-B-Zellen oder Pankreas-A-Zellen, Kardiomyozyten, Milzzellen, Fettzellen, Gliazellen, Fibroblasten, Kupffer'schen Zellen und Krebszellen (vorzugsweise Leukämie-, Lymphom-, Myelom-, Karzinom-, Sarkom- oder Melanomzellen).

## Revendications

1. Processus de production d'une bibliothèque de particules d'AAV recombinantes, le processus comprenant les étapes de :
a) introduction, dans une population de premières cellules hôtes, d'une bibliothèque d'ADN comprenant une pluralité de molécules d'ADN, dans lequel chaque molécule d'ADN de la bibliothèque comprend un génome d'AAV recombinant qui comprend des ITR flanquant un premier gène *cap* d'AAV codant pour des premiers polypeptides Cap, et dans lequel les molécules d'ADN de la bibliothèque diffèrent dans les séquences nucléotidiques de leurs premiers gènes *cap* d'AAV ;
b) culture de la population de premières cellules hôtes dans des conditions telles que :
(i) un second gène cap d'AAV codant pour des seconds polypeptides Cap soit exprimé dans les premières cellules hôtes, dans lequel les seconds polypeptides Cap sont ceux qui confèrent un tropisme aux particules d'AAV encapsidées par de tels polypeptides envers les deuxièmes cellules hôtes,
(ii) un gène *rep* d'AAV et des gènes auxiliaires viraux soient exprimés dans les premières cellules hôtes, et
(iii) les premiers polypeptides Cap d'AAV ne soient pas produits dans les premières cellules hôtes, en raison de l'expression d'un répresseur dans les premières cellules hôtes qui réprime ou empêche l'expression des premiers polypeptides Cap d'AAV ou dans lequel le premier gène *cap* d'AAV est associé fonctionnellement à un promoteur inductible et l'inducteur n'est pas présent dans les premières cellules hôtes,
dans lequel le gène *rep* d'AAV et le second gène cap sont tous deux présents dans le génome d'un adénovirus recombinant qui est présent dans les premières cellules hôtes,
dans lequel des premières particules d'AAV recombinantes sont produites qui sont encapsidées par les seconds polypeptides Cap ;
c) infection d'une population de deuxièmes cellules hôtes avec des premières particules d'AAV recombinantes ;
d) culture de la deuxième population de cellules hôtes dans un milieu de culture dans des conditions telles que :
(i) les premiers polypeptides Cap d'AAV soient produits, et
(ii) un gène *rep* d'AAV et des gènes auxiliaires viraux soient exprimés dans les deuxièmes cellules hôtes,
dans lequel, si le premier gène *cap* d'AAV est associé fonctionnellement à un promoteur inductible, l'inducteur est présent dans la deuxième population de cellules hôtes,
dans lequel le gène *rep* d'AAV est présent dans le génome d'un adénovirus recombinant qui est présent dans la deuxième population de cellules hôtes,
dans lequel une bibliothèque de particules d'AAV de secondes particules d'AAV recombinantes est produite, dans lequel chaque seconde particule d'AAV recombinante de la bibliothèque comprend un génome d'AAV qui comprend des ITR flanquant un premier gène cap d'AAV codant pour un premier polypeptide Cap, dans lequel les particules de la bibliothèque diffèrent dans les séquences nucléotidiques de leurs premiers gènes *cap* d'AAV, et dans lequel chaque particule de la bibliothèque est encapsidée par des polypeptides Cap qui sont codés par le (premier) gène *cap* dans son génome d'AAV ;
et facultativement
e) purification et/ou isolement d'une bibliothèque de secondes particules d'AAV recombinantes à partir des deuxièmes cellules hôtes ou du milieu de culture.

2. Processus selon la revendication 1, dans lequel la bibliothèque d'ADN se présente sous la forme d'une bibliothèque de plasmides ou de vecteurs, de transposons, de molécules d'ADN linéaires ou de particules lentivirales ou de vecteurs lentiviraux.

3. Processus selon la revendication 1 ou la revendication 2, dans lequel les premières et/ou deuxièmes cellules hôtes proviennent d'une lignée cellulaire de production d'AAV ou d'une lignée cellulaire de fabrication d'AAV.

4. Processus selon l'une quelconque des revendications précédentes, dans lequel les premières et/ou deuxièmes cellules hôtes sont sélectionnées dans le groupe consistant en les cellules HEK293, HEK293T, HEK293A, PerC6, 911 et HeLaRC32.

5. Processus selon l'une quelconque des revendications précédentes, dans lequel le génome d'AAV recombinant comprend en outre un gène rapporteur.

6. Processus selon l'une quelconque des revendications précédentes, dans lequel le répresseur est un ARN antisens, de préférence un petits ARN en épingle à cheveux, un petit ARN interférent ou un miARN.

7. Processus selon la revendication 6, dans lequel le répresseur est un ARN antisens qui se lie à l'UTR 5' ou 3' d'un premier ARNm Cap d'AAV, de préférence à l'UTR 3'.

8. Processus selon l'une quelconque des revendications précédentes, dans lequel le premier gène *cap* d'AAV est associé fonctionnellement à un promoteur répressible, le répresseur est présent dans les premières cellules hôtes, et le répresseur n'est pas présent dans les deuxièmes cellules hôtes.

9. Processus selon l'une quelconque des revendications précédentes, dans lequel :
(i) les premières particules d'AAV recombinantes sont de sérotype 1, 2, 3, ou 6 ; et/ou
(ii) les secondes particules d'AAV recombinantes sont du sérotype 9, ou d'un dérivé de celui-ci.

10. Processus selon l'une quelconque des revendications précédentes, dans lequel l'adénovirus recombinant comprend un promoteur tardif majeur (MLP) répressible et une pluralité de gènes tardifs adénoviraux, dans lequel le MLP comprend un ou plusieurs éléments répresseurs qui sont aptes à réguler ou à commander la transcription des gènes tardifs adénoviraux, et dans lequel un ou plusieurs des éléments répresseurs sont insérés en aval de la boîte TATA du MLP.

11. Processus selon l'une quelconque des revendications précédentes, dans lequel les deuxièmes cellules hôtes sont infectées par des premières particules d'AAV recombinantes à une faible multiplicité d'infection (MOI), de préférence à une MOI inférieure à une particule virale par cellule.

12. Processus selon l'une quelconque des revendications précédentes, dans lequel les seconds polypeptides Cap d'AAV ne sont pas produits dans les deuxièmes cellules hôtes.

13. Processus selon l'une quelconque des revendications précédentes, dans lequel une ou plusieurs des secondes particules recombinantes présentent un tropisme envers des troisièmes cellules hôtes, de préférence une troisième cellule hôte choisie dans le groupe consistant en des neurones (de préférence des neurones rétiniens), des hépatocytes, des cellules musculaires, des cellules souches (de préférence des cellules souches hématopoïétiques, des cellules souches mésenchymateuses, des cellules souches embryonnaires, des cellules souches adipeuses ou des cellules souches pluripotentes induites et leurs dérivés), des cellules immunitaires (de préférence des lymphocytes B ou T, des cellules tueuses naturelles, des monocytes, des macrophages ou des granulocytes), des cellules endothéliales, des cellules cardiovasculaires, des cellules épithéliales, des cellules mésenchymateuses, des cellules B pancréatiques ou des cellules A pancréatiques, des cardiomyocytes, des cellules de la rate, des cellules adipeuses, des cellules gliales, des fibroblastes, des cellules de Kupffer et des cellules cancéreuses (de préférence des cellules de leucémie, de lymphome, de myélome, de carcinome, de sarcome ou de mélanome).
